# DEMANDE DE BREVET EUROPEEN

(11) **EP 2 725 025 A1**
(43) Date de publication de la demande: **30.04.2014**
(21) Numéro de dépôt: 12306334.9
(22) Date de dépôt: 26.10.2012
(51) Int. Cl.: C07D 401/14, C07D 403/12, A61K 31/501, A61P 7/00, A61P 29/00, A61P 37/00

(54) **Dérives de 1H-indole-3-carboxamide et leurs utilisation comme antagonistes du P2Y12**

(71) Demandeur: SANOFI, 75008 Paris (FR)
(72) Inventeur: La désignation de l'inventeur n'a pas encore été déposée
(74) Mandataire: Bouron, Estelle

(57) **Abrégé**

L'invention concerne des composés répondant à la formule (I) : 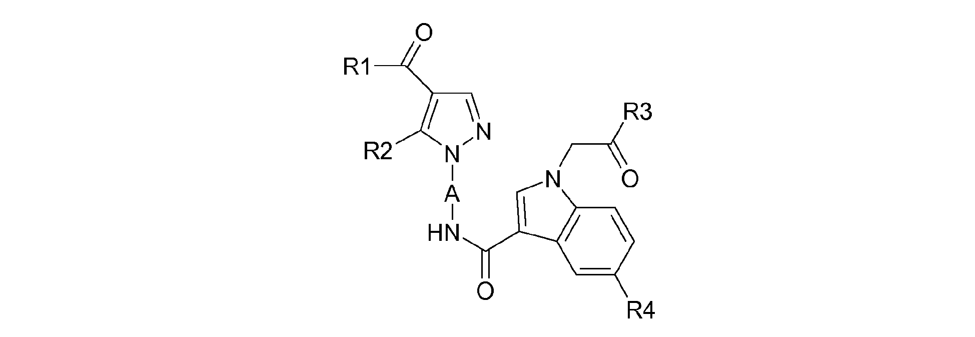 et leur utilisation comme antagonistes du récepteur P2Y12 pour le traitement des maladie cardiovasculaires.

## Description

La présente invention a pour objet de nouveaux dérivés de N-[(1*H*-pyrazol-1-yl)aryl]-1*H*-indole ou 1*H*-indazole-3-carboxamide, leur préparation et leur application en thérapeutique.

Les composés selon la présente invention sont des antagonistes réversibles du récepteur purinergique P2Y12. Ces composés sont des anti-agrégants plaquettaires, exerçant un puissant effet anti-thrombotique. Ils peuvent être utilisés pour le traitement et la prophylaxie de troubles cardiovasculaires tels que les maladies thromboemboliques ou les resténoses.

Dans le monde industrialisé, les complications médicales liées à la survenue d'une thrombose représentent une des principales causes de mortalité. Quelques exemples de pathologies associées au développement d'une thrombose incluent l'infarctus aigu du myocarde, l'angine (de poitrine) instable et l'angine stable chronique, les attaques ischémiques passagères, les accidents vasculaires cérébraux, la maladie vasculaire périphérique, la pré-éclampsie et l'éclampsie, la thrombose veineuse profonde, les embolies (cérébrale, pulmonaire, coronarienne, rénale ...), la coagulation intravasculaire disséminée, ou le purpura thrombotique thrombocytopénique. Il existe également des risques de complications thrombotiques et resténotiques pendant et à la suite de procédures chirurgicales invasives, telles que l'angioplastie, l'endartériectomie carotidienne, le pontage aorto-coronarien par greffe, ou encore le placement de stents ou de prothèses endovasculaires.

Les thromboses artérielles peuvent survenir à la suite d'une lésion de la paroi d'un vaisseau ou d'une rupture d'une plaque d'athérosclérose. Les plaquettes jouent un rôle essentiel dans la formation de ces thromboses. Les plaquettes peuvent être activées soit par des médiateurs libérés dans la circulation sanguine par des cellules circulantes ou par des cellules endothéliales endommagées présentes le long de la paroi du vaisseau, soit par des molécules thrombogènes de la matrice sous-endothéliale - comme le collagène - exposées lors de lésions vasculaires. En outre, les plaquettes peuvent également être activées dans des conditions de flux sanguin à contrainte de cisaillement élevée que l'on retrouve dans des vaisseaux sténosés. Après activation, les plaquettes circulantes adhèrent et s'accumulent au niveau de la lésion vasculaire pour former un thrombus. Pendant ce processus, les thrombi générés peuvent être suffisamment volumineux pour bloquer en partie ou complètement la circulation sanguine dans le vaisseau.

Dans les veines, des thrombi peuvent aussi se former au niveau de zones de stase ou de circulation sanguine ralentie. De par leur nature, ces thrombi veineux peuvent produire des emboles qui se déplacent dans le système vasculaire. Ces emboles sont alors capables de bloquer la circulation sanguine dans des vaisseaux plus distants, tels que les artères pulmonaire ou coronaire.

De nombreuses études ont démontré que l'adénosine 5'-diphosphate (ADP) est un médiateur capital de l'activation et de l'agrégation plaquettaire, jouant un rôle clef dans l'initiation et la progression de la formation d'un thrombus (Maffrand et al, Thromb. Haemostas. (1988) 59, 225-230; Herbert et al, Arterioscl. Thromb. (1993) 13, 1171-1179).

L'ADP est libéré dans la circulation par les globules rouges lésés et les cellules endothéliales de la paroi athéroscléreuse, et plus spécifiquement sécrété par les plaquettes activées où l'ADP est stocké dans les granules denses à très forte concentration. L'agrégation plaquettaire induite à l'ADP est déclenchée par sa liaison à deux récepteurs purinergiques spécifiques, exprimés sur la membrane cellulaire des plaquettes humaines: P2Y1 et P2Y12. Le récepteur P2Y1, couplé à la stimulation de la PLCβ via Gαq, est responsable de la mobilisation des stocks internes de calcium, du changement de forme des plaquettes, et d'une agrégation transitoire à l'ADP. Le récepteur P2Y12, couplé à l'inhibition de l'adénylcyclase via Gαi2 et à l'activation de la PI-3 kinase, est responsable de l'amplification des réponses et de la stabilisation de l'agrégation (Gachet, Thromb. Haemost. (2001) 86, 222-232; Andre et al, J. Clin. Invest. (2003) 112, 398-406). L'utilisation de souris transgéniques P2Y1-/- (Gachet et al, J Clin Invest (1999) 104, 1731-1737; Gachet et al, Circulation (2001) 103, 718-723 ; Gachet et al, Haematologia (2002) 87, 23) et P2YI2-/- (Conley et al, Nature (2001) 409, 202-207) a permis de mettre en évidence l'importance de ces deux récepteurs dans le développement des thromboses *in vivo.* Chez l'Homme, des déficits génétiques sur P2Y12 ont été décrits comme étant associés à un phénotype hémorragique et à une altération prononcée de l'agrégation plaquettaire induite à l'ADP (Kanakura et al, J Thromb Haemost. (2005) 3, 2315-2323).

L'utilisation du Clopidogrel en clinique humaine a fourni la preuve que le blocage du récepteur P2Y12 par un antagoniste représente une stratégie thérapeutique clef dans le traitement des maladies cardiovasculaires. Le Clopidogrel est une pro-drogue de la famille des thiénopyridines dont le métabolite actif se lie de façon covalente au récepteur P2Y12, conduisant à une inhibition irréversible de l'activité plaquettaire *in vivo.* (Savi et al, Biochem Biophys Res Commun (2001) 283, 379-383 ; Savi et al, Proc Natl Acad Sci (2006) 103, 11069-11074). Cette molécule avait initialement montré son efficacité dans plusieurs essais cliniques en réduisant le risque des accidents cardiovasculaires chez des patients à risque ("A randomised, blinded, trial of clopidogrel versus aspirin in patients at risk of ischaemic events (CAPRIE)" CAPRIE steering committee Lancet (1996) 348, 1329-1339 ; "The Clopidogrel in Unstable Angina to Prevent Recurrent Events (CURE). Effects of Clopidogrel in Addition to Aspirin in Patients with Acute Coronary Syndromes without ST-Segment Elevation" CURE steering committee N Engl J Med (2001) 345, 7, 494-502).

Des antagonistes synthétiques du récepteur P2Y12 qui présentent une activité anti-plaquettaire et anti-thrombotique ont été décrits. Néanmoins, le besoin de nouveaux antagonistes possédant des propriétés supérieures subsiste, notamment le besoin d'antagonistes réversibles présentant un meilleur rapport bénéfice sur risque.

La présente invention a pour objet des composés répondant à la formule (I) : 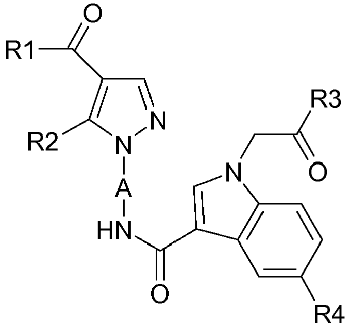 dans laquelle :
A représente un radical bivalent choisi parmi : 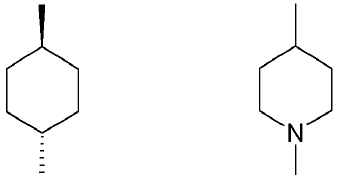
R₁ représente un (C₁-C₄)alkyle;
R₂ représente un (C₁-C₃)alkyle;
R₃ représente un groupe -NR₇R₈, R₇ et R₈, ensemble avec l'atome d'azote auquel ils sont liés constituent un hétérocycle saturé, comprenant de 4 à 6 chainons et pouvant contenir un autre atome d'azote ; ledit hétérocycle étant substitué par au moins un (C₁-C₃)alkyle non-substitué ou substitué par au moins un des groupes suivants:
   - un, deux ou trois atomes d'halogène ou,
   - un (C₁-C₃)alcoxy non-substitué ou substitué par un, deux ou trois atomes d'halogène;
   R₄ représente un atome d'halogène.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques font partie de l'invention.

Les composés de formule (I) peuvent exister à l'état de bases ou salifiés par des acides ou des bases, notamment des acides ou des bases pharmaceutiquement acceptables. De tels sels d'addition font parties de l'invention.

Ces sels sont avantageusement préparés avec des acides ou des bases pharmaceutiquement acceptables, mais les sels d'autres acides ou bases utiles, par exemple, pour la purification ou l'isolement des composés de formule (I), font également partie de l'invention.

Par atome d'halogène, on entend un atome de brome, de chlore, de fluor ou d'iode.

Par alkyle, on entend un radical alkyle linéaire ou ramifié de un à quatre atomes de carbone, tel que le radical méthyle, éthyle, propyle, isopropyle, butyle, isobutyle, sec-butyle, tert-butyle.

Par alcoxy, on entend un radical alcoxy linéaire ou ramifié de un à trois atomes de carbone, tel que le radical méthoxy, éthoxy, propoxy, isopropoxy, butoxy.

Par hétérocycle, on entend un groupe alkyle cyclique comprenant de 4 à 6 atomes formance ce cycle, et dont un ou deux sont des hétéroatomes choisis. On peut notamment citer les groupes pyrolidine, imidazoline, pipéridine, pyrazolidine et pipérazine.

Parmi les composés de formule (I) selon l'invention, on peut citer un sous-groupe de composés dans lequel A représente :

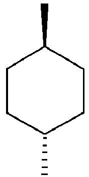

Un autre sous-groupe de composés de formule (I) est tel que A représente :

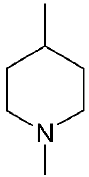

Un autre sous-groupe de composés de formule (I) est tel que R₁ représente un groupe n-propyle.

Un autre sous-groupe de composés de formule (I) est tel que R₃ représente un groupe pipérazine substitué par au moins un (C₁-C₃) alkyle non-substitué ou substitué par au moins un des groupes suivants:
- un, deux ou trois atomes d'halogène ou,
- un (C₁-C₃) alcoxy non-substitué ou substitué par un, deux ou trois atomes d'halogène

Un autre sous-groupe de composés de formule (I) est tel que R₄ représente un atome de chlore.

Les sous-groupes ci-dessus pris séparemment ou en combinaison font également partie de l'invention.

Parmi les composés de formule (I), objets de l'invention, on peut notamment citer les composés suivants :
- 5-Chloro-1-[2-(4-methyl-piperazin-1-yl)-2-oxo-ethyl]-1H-indole-3-carboxylic acid [4-(4-butyryl-5-methyl-pyrazol-1-yl)-cyclohexyl]-amide
- 5-Chloro-1-[2-(4-methyl-piperazin-1-yl)-2-oxo-ethyl]-1H-indole-3-carboxylic acid [4-(4-butyryl-5-methyl-pyrazol-1-yl)-piperidin-1-yl]-amide
- 5-Chloro-1-{2-oxo-2-[4-(3,3,3-trifluoro-propyl)-piperazin-1-yl]-ethyl}-1H-indole-3-carboxylic acid [4-(4-butyryl-5-methyl-pyrazol-1-yl)-cyclohexyl]-amide
- 5-Chloro-1-{2-[4-(2-methoxy-ethyl)-piperazin-1-yl]-2-oxo-ethyl}-1H-indole-3-carboxylic acid [4-(4-butyryl-5-methyl-pyrazol-1-yl)-cyclohexyl]-amide
- 5-Chloro-1-{2-[4-(2-methoxy-ethyl)-piperazin-1-yl]-2-oxo-ethyl}-1H-indole-3-carboxylic acid [4-(4-butyryl-5-methyl-pyrazol-1-yl)-piperidin-1-yl]-amide
- 5-Chloro-1-{2-oxo-2-[4-(2-trifluoromethoxy-ethyl)-piperazin-1-yl]-ethyl}-1H-indole-3-carboxylic acid [4-(4-butyryl-5-methyl-pyrazol-1-yl)-piperidin-1-yl]-amide à l'état de base ou de sel d'addition à un acide ou à une base.

Il est à noter que les composés ci-dessus ont été nommés en nomenclature IUPAC par l'intermédiaire du logiciel AutoNom (Beilstein Informations system).

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans "Protective Group in Organic Synthesis", Green et al., 4th Edition, John Wiley & Sons, Inc., New York, 2007.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans "Advanced Organic Chemistry", M.B. Smith and J. March, 6th Edition, Wiley Interscience, 2007, p. 496-501.

Conformément à l'invention, on peut préparer les composés de formule (I) selon un procédé qui est caractérisé en ce que :
on fait réagir un composé de formule (IIB) : 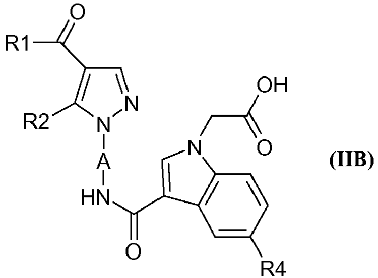 dans laquelle A, R₁, R₂ et R₄ sont tels que définis pour un composé de formule (I) avec une amine de formule (III) :

   HR₃ (**III**)
dans laquelle R₃ est tel que défini pour un composé de formule (I).

Eventuellement, on transforme le composé de formule (I) en l'un de ses sels avec des acides minéraux ou organiques.

La réaction s'effectue en présence d'une base comme, par exemple, la triéthylamine, la 4-diméthylaminopyridine, la N,N-diisopropyléthylamine, la N-méthylmorpholine, la N-éthylmorpholine ou la pyridine et en présence d'un agent de couplage comme, par exemple, le chlorure de bis(2-oxo-1,3-oxazolidin-3-yl)phosphinique, le chloroformate d'isobutyle, le 1,1'-carbonyldiimidazole, le chlorohydrate de 1-(3-diméthylaminopropyl)-3-éthylcarbodiimide, le N-Hydroxybenzotriazole, le 2-cyano-2-(hydroxyimino)acétate d'éthyle. Le solvant utilisé est, par exemple, le DCM, le 1,2-dichloroéthane ou le N,N-diméthylformamide. La température de la réaction est comprise entre -10°C et la température de reflux du solvant.

Les composés de formule (I) peuvent également se préparer par réaction d'un acide ou d'un dérivé fonctionnel activé de cet acide de formule : 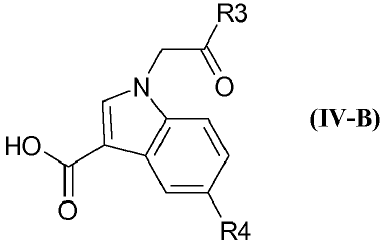 dans laquelle R₃ et R₄ sont tels que définis pour un composé de formule (I), avec un composé de formule : 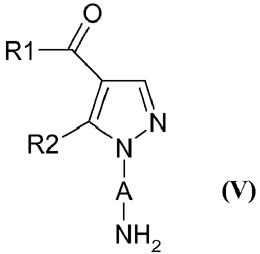 dans laquelle A, R₁ et R₂ sont tels que définis pour un composé de formule (I).

Lorsqu'on traite un composé de formule (V) avec l'acide de formule (IV-B) lui-même, on opère en présence d'un agent de couplage utilisé en chimie peptidique tel que le 1,3-dicyclohexylcarbodiimide ou le 1-ethyl-3-(3-dimethylaminopropyl)carbodiimide hydrochloride ou l'hexafluorophosphate de benzotriazol-1-yl-oxytris(diméthylamino)phosphonium ou le N-hydroxybenzotriazole ou l'hexafluorophosphate de benzotriazol-1-yl-oxytris(pyrrolidino)phosphonium ou le tétrafluoroborate de 2-(1*H*-benzotriazol-1-yl)-1,1,3,3-tétraméthyluronium ou le chlorhydrate de 1-(3-diméthylaminopropyl)-3-éthylcarbodiimide, en présence d'une base telle que la triéthylamine, la N,N-diisopropyléthylamine ou la 4-diméthylaminopyridine, dans un solvant tel que le DCM, le 1,2-dichloroéthane, le N-N-diméthylformamide ou le tétrahydrofurane à une température comprise entre -10°C et la température de reflux du solvant.

Comme dérivé fonctionnel activé de l'acide (IV-B) on peut utiliser le chlorure d'acide, l'anhydride, un anhydride mixte, un ester alkylique en C₁-C₄ dans lequel l'alkyle est linéaire ou ramifié, un ester activé, par exemple l'ester dep-nitrophényle.

Ainsi, on peut, par exemple, faire réagir le chlorure de l'acide obtenu par réaction du chlorure de thionyle ou du chlorure d'oxalyle sur l'acide de formule (IV-B), avec le composé de formule (V), dans un solvant, tel qu'un solvant chloré (le DCM, le 1,2-dichloroéthane, le chloroforme par exemple), un éther (tétrahydrofurane, dioxane par exemple), un amide (N,N-diméthylformamide par exemple) ou la pyridine, sous une atmosphère inerte, à une température comprise entre 0°C et la température de reflux du solvant, en présence d'une amine tertiaire telle que la triéthylamine, la N-méthylmorpholine, la pyridine, la 4-diméthylaminopyridine ou le 1,8-diazabicyclo[5.4.0]undec-7-ène.

De façon particulière, on peut préparer certains composés de formule (I) à partir d'autres composés de formule (I).

Les composés de formule (I) ainsi obtenus peuvent être ultérieurement séparés du milieu réactionnel et purifiés selon les méthodes classiques, par exemple, par cristallisation ou chromatographie sur colonne de silice.

Les composés de formule (I) ainsi obtenus sont isolés sous forme de base libre ou de sel, selon les techniques classiques.

Les composés de formule (IIB) peuvent se préparer selon un procédé dans lequel on hydrolyse, en milieu acide ou basique, un composé de formule (IIA) : 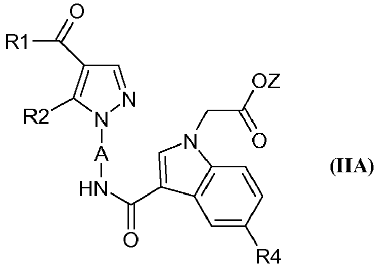 dans laquelle A, R₁, R₂ et R₄ sont tels que définis pour un composé de formule (I) et Z représente un (C₁-C₄) alkyle.

Eventuellement, on transforme le composé de formule (IIA) en l'un de ses sels avec des bases minérales ou organiques.

La réaction s'effectue en milieu acide par action d'un acide fort, comme, par exemple, l'acide chlorhydrique ou l'acide sulfurique, dans un solvant, en particulier un solvant polaire, tel que, par exemple, le dioxane ou l'eau et à une température comprise entre -10°C et 110°C.

La réaction s'effectue en milieu basique par action d'une base alcaline, comme, par exemple, l'hydroxyde de potassium, l'hydroxyde de lithium ou l'hydroxyde de sodium, dans un solvant, en particulier un solvant polaire, tel que, par exemple, le dioxane, le tétrahydrofurane, l'eau, le méthanol, l'éthanol ou un mélange de ces solvants et à une température comprise entre -10°C et la température de reflux du solvant.

Les composés de formule (IIA) peuvent se préparer par réaction d'un acide ou d'un dérivé fonctionnel activé de cet acide de formule : 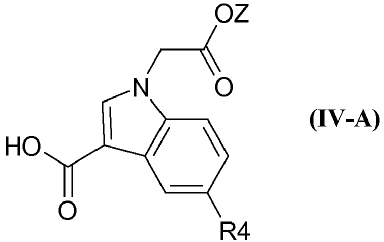 dans laquelle R₄ est tel que défini pour un composé de formule (I) et Z représente un (C₁-C₄)alkyle, avec un composé de formule : 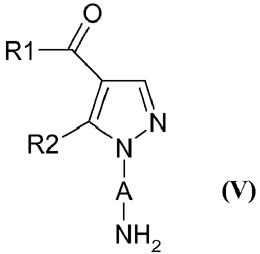 dans laquelle A, R₁ et R₂ sont tels que définis pour un composé de formule (I).

Lorsqu'on traite un composé de formule (V) avec l'acide de formule (IV-A) lui-même, on opère en présence d'un agent de couplage utilisé en chimie peptidique tel que le 1,3-dicyclohexylcarbodiimide ou le 1-ethyl-3-(3-dimethylaminopropyl)carbodiimide hydrochloride ou l'hexafluorophosphate de benzotriazol-1-yl-oxytris(diméthylamino)phosphonium ou l'hexafluorophosphate de benzotriazol-1-yl-oxytris(pyrrolidino)phosphonium ou le tétrafluoroborate de 2-(1*H-*benzotriazol-1-yl)-1,1,3,3-tétraméthyluronium ou le chlorhydrate de 1-(3-diméthylaminopropyl)-3-éthylcarbodiimide, en présence d'une base telle que la triéthylamine, la N,N-diisopropyléthylamine ou la 4-diméthylaminopyridine, dans un solvant tel que le DCM, le 1,2-dichloroéthane, le N-N-diméthylformamide ou le tétrahydrofurane à une température comprise entre -10°C et la température de reflux du solvant.

Comme dérivé fonctionnel activé de l'acide (IV-A) on peut utiliser le chlorure d'acide, l'anhydride, un anhydride mixte, un ester alkylique en C₁-C₄ dans lequel l'alkyle est linéaire ou ramifié, un ester activé, par exemple l'ester de *p*-nitrophényle.

Ainsi, on peut, par exemple, faire réagir le chlorure de l'acide obtenu par réaction du chlorure de thionyle ou du chlorure d'oxalyle sur l'acide de formule (IV-A), avec le composé de formule (V), dans un solvant, tel qu'un solvant chloré (le DCM, le 1,2-dichloroéthane, le chloroforme par exemple), un éther (tétrahydrofurane, dioxane par exemple), un amide (N,N-diméthylformamide par exemple) ou la pyridine, sous une atmosphère inerte, à une température comprise entre 0°C et la température de reflux du solvant, en présence d'une amine tertiaire telle que la triéthylamine, la N-méthylmorpholine, la pyridine, la 4-diméthylaminopyridine ou le 1,8-diazabicyclo[5.4.0]undec-7-ène.

Les composés de formule (III) peuvent être connus, commercialisés ou se préparer selon des méthodes connues de l'homme de l'art.

Les composés de formule (V) se préparent par réaction de déprotection d'un composé de formule : 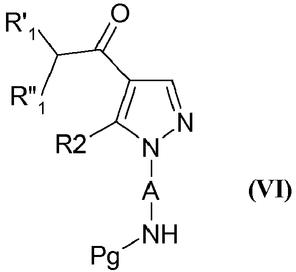 dans laquelle A et R₂ sont tels que définis pour un composé de formule (I), R'₁ représente un atome d'hydrogène ou un (C₁-C₃)alkyle, R"1 représente un atome d'hydrogène ou un groupe -COOZ. Z représente un (C₁-C₄) alkyle et Pg représente un groupe protecteur, de préférence un groupe *tert*-butoxycarbonyle ou benzyloxycarbonyle.

La réaction s'effectue par action d'un acide comme, par exemple, l'acide chlorhydrique ou l'acide trifluoroacétique en présence ou non d'un solvant, par exemple l'eau ou le dioxane, et à une température comprise entre la température ambiante et la température de reflux du milieu réactionnel.

Les composés de formule (VI) pour lesquels R"₁ = H, se préparent par réaction d'un composé de formule : 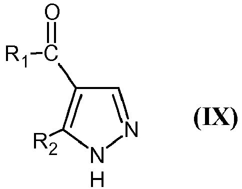 dans laquelle R₁ et R₂ sont tels que définis pour un composé de formule (I), avec un composé de formule :

Y-A-NHPg (**X**)

dans laquelle A est tel que défini pour un composé de formule (I), Y représente un groupe partant tel qu'un atome d'halogène, un méthanesulfonate, un benzène sulfonate, un p-toluènesulfonate, un triflate ou un acétate et Pg représente un groupe protecteur, de préférence un groupe tert-butoxycarbonyle ou benzyloxycarbonyle.

La réaction s'effectue en présence d'une base comme, par exemple, le carbonate de potassium, le carbonate de césium ou le tert-butylate de potassium. La réaction s'effectue dans un solvant tel que, par exemple, le diméthylsulfoxyde, le N,N-diméthylformamide, le dioxane ou le tétrahydrofurane et à une température comprise entre 0°C et 150°C.

Les composés de formule (VI) pour lesquel R"₁ = COOZ peuvent se préparer selon le SCHEMA 1 ci-après, dans lequel R₂ est tel que défini pour un composé de formule (I), Z représente un (C₁-C₄) alkyle et Pg représente un groupe protecteur, de préférence un groupe *tert*-butoxycarbonyle ou benzyloxycarbonyle.

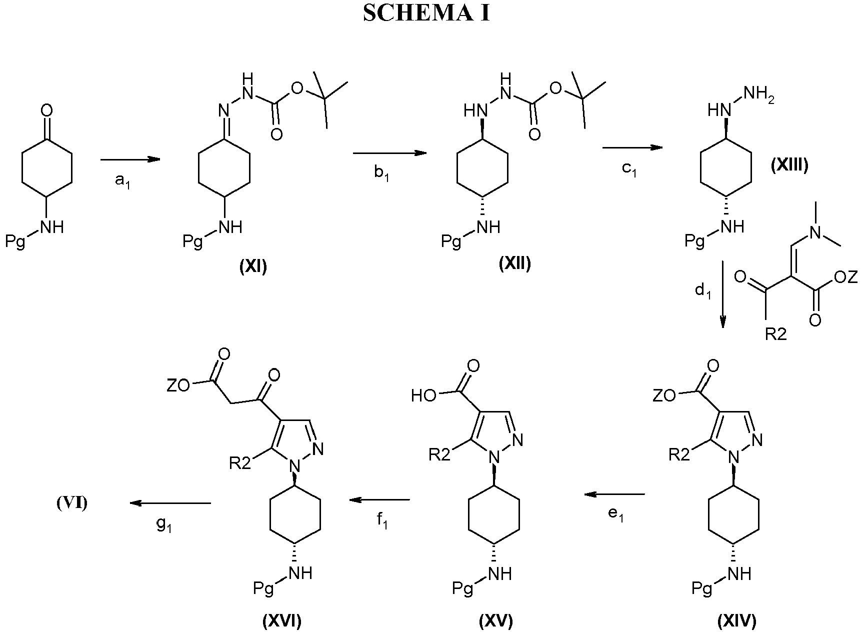

A l'étape a₁ du SCHEMA I, on condense une 4-amino-cyclohexanone protégée avec le carbazate de *tert*-butyle pour obtenir le composé (XI).

A l'étape b₁, on réduit le composé (XI) à l'aide de cyanoborohydrure de sodium dans le méthanol, puis sépare l'isomère de conformation trans (XII) par cristallisation dans l'acétate d'éthyle en présence d'une quantité stoechiométrique d'acide chlorhydrique.

A l'étape c₁, on déprotège la fonction hydrazine en présence d'acide chlorhydrique en excès dans le dioxanne pour obtenir le composé (XIII).

A l'étape d₁, on condense le dérivé hydrazine (XIII) avec du 2-[1-Dimethylamino-methylidene]-3-oxo-butyric acid ethyl ester en présence de triéthylamine dans l'éthanol à reflux afin de générer le dérivé pyrazole (XIV).

A l'étape e₁, on hydrolyse le composé de formule (XIV) en présence d'hydroxide de sodium aqueux dans un mélange de tétrahydrofurane et d'éthanol pour obtenir l'acide carboxylique correspondant (XV).

A l'étape f₁, on fait réagir le composé de formule (XV) avec du 1,1'-carbonyldiimidazole puis, sans isoler, on traite le composé intermédiaire ainsi obtenu avec un sel de magnésium d'un hémi-ester de l'acide malonique selon la méthode décrite dans Angew. Chem. Int. Ed. Engl (1979) 18(1), 72-74.

A l'étape g₁, on alkyle le composé de formule (XVI) ainsi obtenu par réaction d'un halogénure, d'un mésylate ou d'un tosylate de (C₁-C₃)alkyle, en présence d'une base telle que, par exemple, le carbonate de potassium, d'un catalyseur de transfert de phase tel que, par exemple, le bromure de tétrabutylammonium dans un solvant comme, par exemple, le tétrahydrofurane et à une température comprise entre 0°C et la température de reflux du solvant.

Les composés de formule (IX) dans laquelle R₁ = (C₁-C₄)alkyle peuvent se préparer selon le SCHEMA II ci-après dans lequel R₂ est tel que défini pour un composé de formule (I), Pg représente un groupe protecteur, de préférence un groupe trityle.

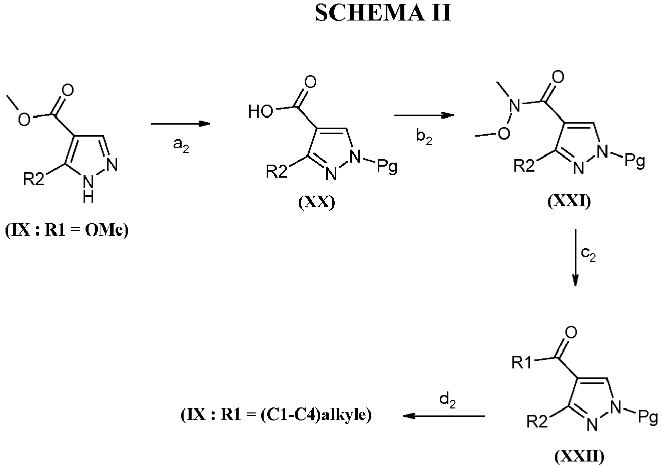

A l'étape a₂ du SCHEMA II, on protège l'atome d'azote du composé de formule (IX) pour lequel R₁ = OMe, en particulier par un groupe trityle, puis hydrolyse en milieu basique le composé intermédiaire obtenu.

A l'étape b₂, le composé de formule (XX) ainsi obtenu est mis en réaction avec la N-méthoxyméthanamine, en présence d'un agent de couplage tel que, par exemple, le chlorure de bis(2-oxo-1,3-oxazolidin-3-yl)phosphinique et en présence d'une base telle que, par exemple, la 4-diméthylaminopyridine. La réaction s'effectue dans un solvant comme, par exemple, le dichlorométhane et à une température comprise entre 0°C et la température ambiante.

Le composé de formule (XXI) ainsi obtenu est mis à réagir à l'étape c₂ avec un composé organométallique tel qu'un halogénure de (C₁-C₄)alkylmagnésium, dans un solvant tel que l'éther diéthylique ou le tétrahydrofurane et à une température comprise entre -70°C et la température ambiante.

Le composé de formule (XXII) ainsi obtenu est déprotégé à l'étape d₂ selon les méthodes classiques (Protective Group in Organic Synthesis, Green et al., 4th Edition, John Wilev & Sons. Inc.. New York. 20071.

Les composés de formule (X) dans lesquels A = 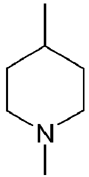 peuvent se préparer selon le SCHEMA III ci-après dans lequel Y représente un mésylate et Pg représente un groupe protecteur, de préférence un groupe tert-butoxycarbonyle.

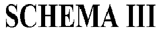

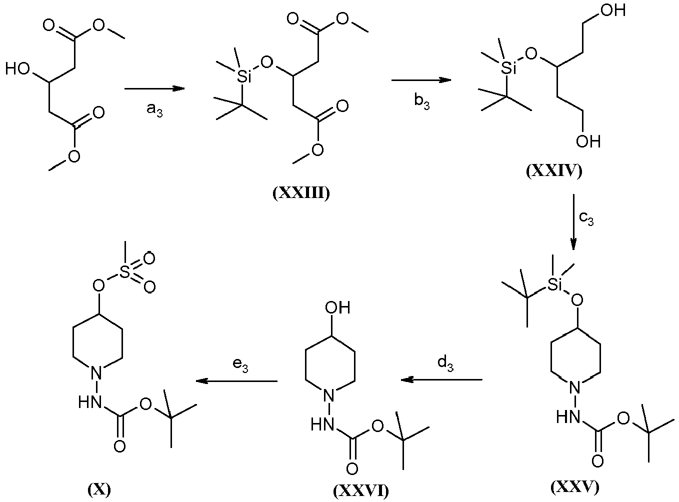

A l'étape a₃ du SCHEMA III, on protège l'alcool secondaire du diméthyl 3-hydroxyglutarate par un groupement *tert*-butyldiméthylsilyle.

A l'étape b₃, on réduit le composé (XXIII) ainsi obtenu en présence de borohydrure de lithium dans le diéthyléther à 0°C pour générer le composé (XXIV).

A l'étape c₃, on oxyde les deux fonctions alcools en aldéhyde par une oxydation de Swern puis génère le composé (XXV) par double amination réductrice cyclisante avec le carbazate de *tert*-butyle en présence de triacétoxyborohydrure de sodium.

A l'étape d₃, on déprotège la fonction sylilée afin de régénérer l'alcool secondaire du composé (XXVI).

A l'étape e₃, on active l'alcool en présence de chlorure de méthanesulfonyle, de triéthylamine et de 4-diméthylaminopyridine, dans le dichlorométhane pour obtenir le mésylate (X).

Les composés de formule (IV-A) pour lesquels R₃ = OZ se préparent par réaction d'un composé de formule: 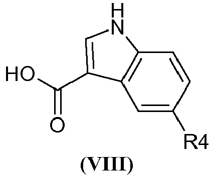 dans laquelle R₄ est tel que défini pour un composé de formule (I), avec un composé de formule (VII) : 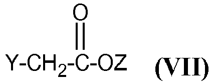 dans laquelle Y représente un groupe partant tel qu'un atome d'halogène, un méthanesulfonate, un benzènesulfonate, un p-toluènesulfonate ou un triflate et Z représente un (C₁-C₄)alkyle.

La réaction s'effectue en présence de deux équivalents d'une base forte comme, par exemple, l'hydrure de sodium, dans un solvant, en particulier un solvant polaire aprotique, comme, par exemple, le N,N-diméthylformamide et à une température comprise entre -30°C et la température de reflux du solvant.

Les composés de formule (IV-B) se préparent selon le schéma IV ci-après dans laquelle R₄ est tel que défini pour un composé de formule (I) :

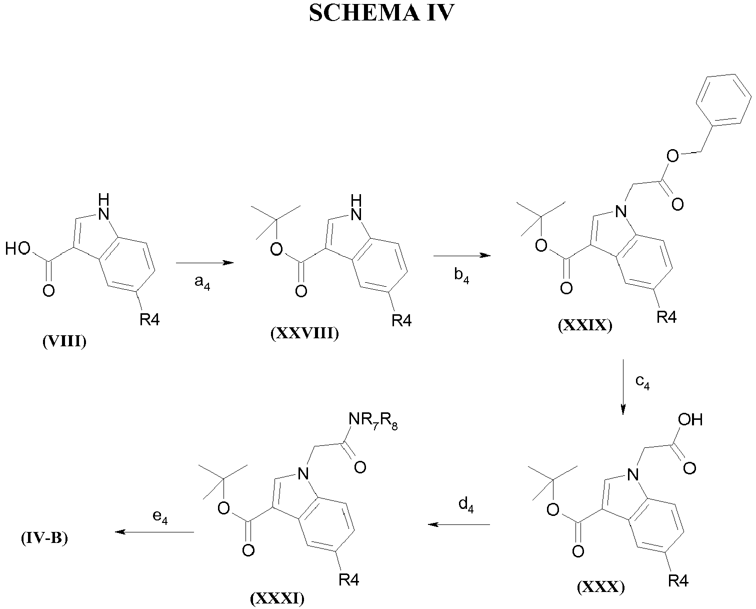

A l'étape a₄ du SCHEMA IV, on protège l'acide carboxylique du dérivé 3-carboxyindole (VIII) en présence de N,N-diméthylformamide di-*tert*-butyl acetal dans le benzène afin de générer l'ester tert-butylique (XXVIII) correspondant.

A l'étape b₄, on alkyle le composé (XXVIII) ainsi obtenu à l'aide d'un composé de formule (VII) ci-dessous, 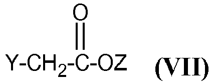 dans laquelle Y représente un groupe partant tel qu'un atome d'halogène, un méthanesulfonate, un benzènesulfonate, un p-toluènesulfonate ou un triflate et Z représente un (C₁-C₄)alkyle ou un groupement benzyle, en présence d'une base telle que le carbonate de césium et d'un solvant, tel que le diméthylformamide.

A l'étape c₄, on hydrolyse l'ester du composé (XXIX) en présence d'hydroxide de lithium à 0°C pour obtenir le composé (XXX).

A l'étape d₄, on génère le lien carboxamide dans des conditions d'activation avec le couple 1-(3-diméthylaminopropyl)-3-éthylcarbodiimide /pentafluorophenol en présence de N-éthylmorpholine et de l'amine HR₃ désiré pour conduire au composé (XXXI).

A l'étape e₄, on déprotège l'ester *tert*-butylique à l'aide d'acide trifluoroacétique dans le dichlorométhane permettant de générer le composé (IV-B).

Les composés de formule (VII) sont connus, commercialisés ou préparés selon des méthodes connues.

Les composés de formule (VIII) peuvent être commerciaux ou se préparer selon des adaptations de méthodes décrites dans la littérature telles que, par exemple, celles représentées au schéma V dans lequel R4 est tel que défini pour un composé de formule (I) :

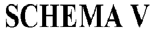

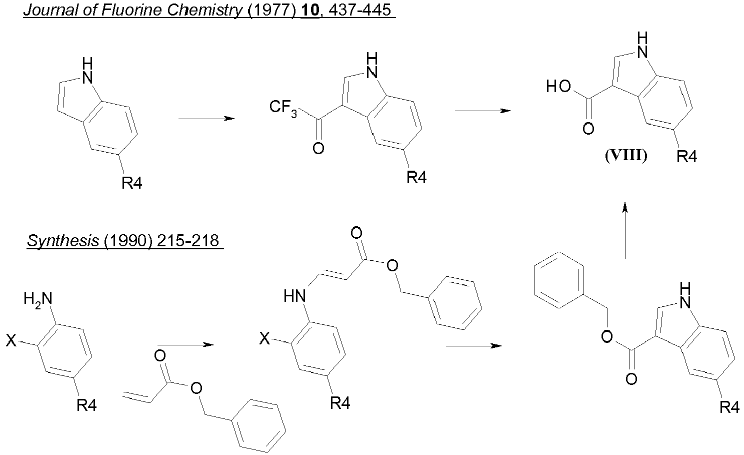

Les composés de formule (IX) dans laquelle R₁ = (C₁-C₄)alcoxy sont connus, commercialisés ou préparés selon des méthodes connues (Synlett (2004) 4, 703-707).

L'invention, selon un autre de ses aspects, a également pour objet les composés nouveaux de formule (IIA). Ces composés sont utiles comme intermédiaire de synthèse des composés de formule (I).

L'invention a également pour objet les composés nouveaux de formule (IIB). Ces composés sont utiles comme intermédiaire de synthèse des composés de formule (I).

L'invention, selon un autre de ses aspects, a également pour objet l'utilisation des composés de formule (I), tels quels ou sous forme radiomarquée comme outils pharmacologiques chez l'homme ou chez l'animal, pour la détection et le marquage du récepteur purinergique P2Y12.

Les exemples suivants décrivent la préparation de certains composés conformes à l'invention. Ces exemples ne sont pas limitatifs et ne font qu'illustrer la présente invention.

Dans les Préparations et dans les Exemples on utilise les abréviations suivantes :
Me : méthyle
Et : éthyle
n-Pr : n-propyle
Ph : phényle éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
DMAP : 4-diméthylaminopyridine
DIPEA : diisopropyléthylamine
HOAT : 1-hydroxy-7-azabenzotriazole
HOBT : 1-hydroxybenzotriazole
TFA : acide trifluoroacétique
BOP-Cl : chlorure de bis-(2-oxo-1,3-oxazolidin-3-yl)phosphinique
EDC : chlorhydrate del-(3-diméthylaminopropyl)-3-éthylcarbodiimide
NaOH : hydroxyde de sodium
KOH : hydroxyde de potassium
HCl : acide chlorhydrique
i-PrOH : isopropanol
MeCN : acétonitrile
NaBH₄ : borohydrure de sodium
NaHCO₃ : hydrogénocarbonate de sodium
NaH : hydrure de sodium
Na₂SO₄ : sulfate de sodium
TBAF : tétra-n-buylammonium
UV : ultraviolet
Ether chlorhydrique 1N ou 2N : solution 1N ou 2N d'acide chlorhydrique dans l'éther diéthylique
HCl 1N (ou 2N) dans l'éther : solution 1N (ou 2N) d'acide chlorhydrique dans l'éther diéthylique
HCl 4N dans le dioxane : solution 4N d'acide chlorhydrique dans le dioxane
F : point de fusion
TA : température ambiante
CLHP : chromatographie liquide haute performance
Saumure : solution saturée de chlorure de sodium dans l'eau.

Les spectres de résonance magnétique nucléaire du proton (RMN ¹H) sont enregistrés sur des spectromètres Bruker (250, 400 et 500 MHz) dans le DMSO-d₆. Les déplacements chimiques δ sont exprimés en parties par million (ppm). Pour l'interprétation des spectres, on utilise les abréviations suivantes : s : singulet, d : doublet, t : triplet, q : quadruplet : m : massif, mt : multiplet, se : singulet élargi, dd : doublet dédoublé, br : pic large.

Les composés selon l'invention sont analysés par couplage CLHP-UV-MS (chromatographie liquide/détection UV/détection de masse).

L'appareil utilisé est composé d'une chaine chromatographique équipée d'un détecteur à barrette de diodes et d'un spectromètre de masse quadripôlaire. On mesure le pic moléculaire (MH⁺) et le temps de rétention (tR) en minutes.
Méthode A : Phenomenex Luna C18(2) column 10x2 mm, 3 µm
Solvant A : eau + 0,05% TFA
Solvant B : MeCN
1.1 ml/min, 30 °C; Agilent series 1100 MSD

| Gradient (minutes) | A | B |
|---|---|---|
| 0 | 93 | 7 |
| 1.2 | 5 | 95 |
| 1.4 | 5 | 95 |

Methode B: Waters XBridge C18 4,6 x 50 mm, 2,5 µm,
Solvant A : eau + 0,1 % d'acide formique
Solvant B : MeCN + 0,08% d'acide formique
1,3 ml/min ; 20 °C ; Waters Ultima Triple Quad MS

| Gradient (minutes) | A | B |
|---|---|---|
| 0 | 97 | 3 |
| 3.5 | 40 | 60 |
| 4 | 2 | 98 |
| 5 | 2 | 98 |
| 5.2 | 97 | 3 |
| 6.5 | 97 | 3 |

Methode C: Merck Chromo lith C18 2x50 mm; 2.5 µm.
Solvant A : eau + 0,05% TFA
Solvant B : MeCN + 0,05% TFA
2.4 ml/min ; 20 °C ; Tecan LCT

| Gradient (minutes) | A | B |
|---|---|---|
| 0 | 98 | 2 |
| 0.2 | 98 | 2 |
| 2.4 | 2 | 98 |
| 3.2 | 2 | 98 |
| 4 | 98 | 2 |

Méthode D : Agilent 1100 series. Symmetry C18 3,5µm (2,1 x 50 mm, Waters)
Solvant A : eau + 0,005% TFA
Solvant B : MeCN + 0,005% TFA
0,4ml/min, 25°C ; MSD SL (Agilent) ESI⁺.

| Gradient (minutes) | A | B |
|---|---|---|
| 0 | 100 | 0 |
| 10 | 0 | 100 |
| 15 | 0 | 100 |

L'enregistrement des spectres de masse est effectué en mode électrospray (ESI) positif, afin d'observer les ions issus de la protonation de composés analysés (MH⁺), ou de la formation d'adduits avec d'autres cations tels que Na⁺, K⁺, etc.

### PREPARATIONS

### 1. Préparations des composés de formule (IX)

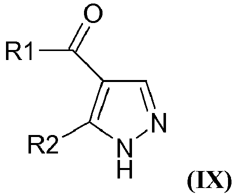

1-(5-méthyl-1*H*-pyrazol-4-yl)butan-1-one.
(IX) : R₁ = n-Pr ; R₂ = Me.

### Etape 1 : acide 3-méthyl-1-trityl-1H-pyrazole-4-carboxylique (XX).

A une solution de 6,60 g de 5-méthyl-1*H* pyrazole-4-carboxylate de méthyle dans 50 mL de DMF, on ajoute 8,46 g de carbonate de potassium et 15,8 g de chlorure de trityle. Après 5 jours à TA, on ajoute de l'AcOEt, lave à l'eau, à la saumure, sèche sur Na₂SO₄ et évapore à sec. Le résidu huileux est mis en suspension dans 100 mL d'un mélange (50/50 ; v/v) d'éthanol et d'eau. On ajoute 9,95 g d'hydroxyde de potassium et chauffe au reflux pendant 6 heures. On filtre le milieu réactionnel à chaud, concentre le filtrat et acidifie avec une solution d' HCl 1N. On filtre le précipité formé, lave avec un mélange (50/50 ; v/v) d'éther iso et d'AcOEt et sèche sous vide. On obtient 9,70 g du composé attendu sous forme d'une poudre blanche.

RMN ¹H : DMSO-d₆ (250 MHz) : δ (ppm) : 2.33 (3H, s) ; 7.03-7.09 (6H, m) ; 7.34-7.43 (9H, m) ; 7.61 (1H, s).

### Etape 2 : N-méthoxy-N,3-diméthyl-1-trityl-1H-pyrazole-4-carboxamide (XXI).

A une solution de 9,70 g du composé obtenu à l'étape précédente, dans 100 mL de DCM, on ajoute 10,3 g de DMAP et 10,3 g de BOP-Cl. On ajoute ensuite par portion 3,85 g de chlorhydrate de N-méthoxyméthanamine et agite pendant 1 heure à TA. On évapore puis reprend le résidu à l'acétate d'éthyle, lave à l'eau, à la saumure, sèche sur Na₂SO₄ puis évapore à sec. On triture à l'éther iso, filtre et sèche à l'étuve à vide. On obtient 10,4 g du composé attendu sous forme d'une poudre blanche.

RMN ¹H : DMSO-d₆ (250 MHz) : δ (ppm) : 2.34 (3H, s) ; 3.13(3H, s) ; 3.39 (3H, s) ; 7.05-7.15 (6H, m) ; 7.33-7.44 (9H, m) ; 7.71 (1H, s).

### Etape 3 : 1-(3-méthyl-1-trityl-1H-pyrazol-4-yl)butan-1-one (XXII).

A une solution de 10,4 g du composé obtenu à l'étape précédente, dans 130 mL de THF à -30°C, on ajoute goutte à goutte, 32,7 mL d'une solution 2M de chlorure de n-propylmagnésium dans l'éther. On revient à TA puis agite pendant 4 heures. On place le milieu réactionnel à -30°C puis ajoute (goutte à goutte au début) 50 mL d'eau. On revient à TA, ajoute 250 mL d'une solution d'HCl 1N puis extrait avec de l'AcOEt. On lave à l'eau, à la saumure, sèche sur Na₂SO₄ puis évapore à sec. On triture dans l'éther iso, filtre et sèche à l'étuve à vide. On obtient 8,4 g du composé attendu sous forme d'une poudre blanche.

RMN ¹H : DMSO-d₆ (400 MHz) : δ (ppm) : 0.84 (3H, t) ; 1.52 (2H, sext) ; 2.34 (3H, s) ; 2.62 (2H, t) ; 7.04-7.12 (6H, m) ; 7.33-7.45 (9H, m) ; 7.93 (1H, s).

### Etape 4 : 1-(5-méthyl-1H-pyrazol-4-yl)butan-1-one.

On agite 8,4 g du composé obtenu à l'étape précédente en suspension, dans 50 mL de HCl 4N dans le dioxane pendant 6 heures. On évapore à sec, triture à l'éther iso, filtre et sèche à l'étuve à vide. On obtient 3,1 g du composé attendu sous forme d'une gomme incolore.

RMN ¹H : DMSO-d₆ (250 MHz) : δ (ppm) : 0.90 (3H, t) ; 1.58 (2H, sext) ; 2.39 (3H, s) ; 2.71 (2H, t) ; 8.15 (1H, s).

### 2. Préparations des composés de formule (V)

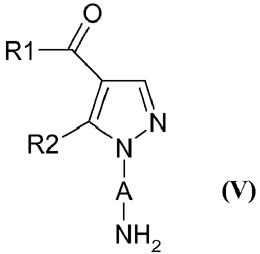

### Préparation 2.1

### 1-[1-(4-Amino-trans-cyclohexyl)-5-methyl-1H-pyrazol-4-yl]-butan-1-one

(V):R₁ = nPr; R₂ = Me_{;} A =

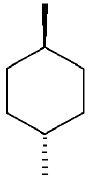

### Etape 1: N'-(4-Benzyloxycarbonylamino-cyclohexylidene)-hydrazinecarboxylic acid tert-butyl ester (XI)

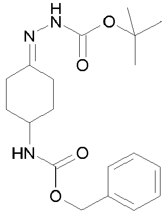

11.3 g (44.3 mmol) de (4-Oxo-cyclohexyl)-carbamate de benzyl et 6.6 g (48.7 mmol) de carbazate de tert-butyle sont agités dans 140 mL de méthanol à TA pendant 4h. Le milieu réactionnel est évaporé à sec. Le résidu solide est trituré à l'éther iso, le précipité est filtré puis séché à l'étuve à vide pour donner 16.4 g de produit attendu sous forme d'une poudre rosée.

Rendement quantitatif.

RMN ¹H : DMSO-d₆ (400 MHz) : δ (ppm) : 9.54 (1H, br) ; 7.43-7.24 (6H, m) ; 5.01 (2H, s) ; 3.60 (2H, m) ; 2.74 (1H, m) ; 2.35-2.11 (2H, m) ; 2.01-1.76 (2H, m) ; 1.47-1.25 (11H, m).

### Etape 2: Chlorhydrate du N'-(4-Benzyloxycarbonylamino-trans-cyclohexyl)-hydrazine-carboxylic acid tert-butyl ester (XII)

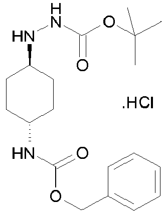

16.3 g (45.1 mmol) de N'-(4-Benzyloxycarbonylamino-cyclohexylidene)-hydrazinecarboxylic acid tert-butyl ester préparé à l'étape précédente sont mis en suspension dans 270 mL d'une solution d'acide acétique/H₂0 (1/1 ; v/v). 2.78 g (44.2 mmol) de cyanoborohydrure de sodium sont ajoutés par portions et le mélange réactionnel est agité à TA pendant la nuit. 140 mL de NaOH à 35% sont ajoutés lentement (pH final = 6-7). Le précipité formé est filtré, lavé à l'eau (3x) puis séché à l'étuve à vide à 60°C (mélange contenant, d'après une analyse RMN 66 % de composé de conformation trans attendu et 34 % de composé de conformation cis).

La poudre blanche obtenue (16.3 g) est solubilisée dans 370 mL d'acétate d'éthyle. 24.7 mL (49.3 mmol) d'une solution de HCl 2N dans le diéthylether sont ajoutés goutte à goutte. Après agitation pendant la nuit, le précipité est filtré, lavé à l'acétone puis séché à l'étuve à vide à 60°C pour obtenir 11.4 g d'une poudre blanche (RMN ¹H : 100% du dérivé de conformation trans).

Rendement = 63%

RMN ¹H : DMSO-d₆ (400 MHz) : δ (ppm) : 10.90 (1H, br) ; 10.18 (1H, br) ; 7.43-7.19 (6H, m) ; 5.01 (2H, s) ; 3.23 (1H, m) ; 3.06 (1H, m) ; 1.96 (2H, m) ; 1.88 (2H, m) ; 1.49-1.31 (11H, m) ; 1.18 (2H, m).

### Etape 3: Chlorhydrate du (4-Hydrazino-trans-cyclohexyl)-carbamic acid benzyl ester (XIII)

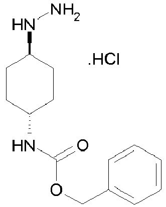

11.3 g (28.3 mmol) du Chlorhydrate du N'-(4-Benzyloxycarbonylamino-*trans-*cyclohexyl)-hydrazine-carboxylic acid tert-butyl ester préparé à l'étape précédente sont solubilisés dans 250 mL de dioxanne. 93.3 mL d'une solution de HCl 4N dans le dioxanne (373 mmol) sont ajoutés goutte à goutte et l'agitation est maintenue pendant 60 h à TA. Le précipité formé est filtré et séché à l'étuve à vide à 60°C pour obtenir 7.61 g de cristaux blancs.

Rendement = 90 %

RMN ¹H : DMSO-d₆ + TFA (250 MHz) : δ (ppm) : 7.34 (5H, m) ; 5.00 (2H, s) ; 3.24 (1H, m) ; 2.84 (1H, m) ; 2.10-1.79 (4H, m) ; 1.22 (4H, m).

### Etape 4: 1-(4-Benzyloxycarbonylamino-trans-cyclohexyl)-5-methyl-1H-pyrazole-4-carboxylic acid ethyl ester (XIV)

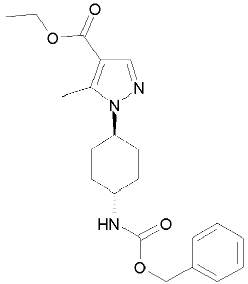

A 6.78 g (22.2 mmol) de Chlorhydrate du (4-Hydrazino-*trans*-cyclohexyl)-carbamic acid benzyl ester préparé à l'étape précédente dans 45 mL d'éthanol sont ajoutés 8.83 mL (63.3 mmol) de triéthylamine. 4.6 g de 2-[1-Dimethylamino-methylidene]-3-oxo-butyric acid ethyl ester sont ajoutés et le milieu réactionnel est porté à reflux pendant 4h. Le milieu réactionnel est évaporé à sec. Le résidu est repris avec un mélange H₂O/AcOEt. La phase organique est collectée et la phase aqueuse est extraite à l'acétate d'éthyle (3x). Les phases organiques rassemblées sont lavées à l'eau (3x), à la saumure (1x), séchées sur Na₂SO₄ puis évaporées à sec. Le résidu est trituré à l'éther iso, filtré puis séché à l'étuve à 60°C pour obtenir 6.0 g de produit attendu sous forme d'une poudre beige.

Rendement = 74%.

RMN ¹H : DMSO-d₆ (400 MHz) : δ (ppm) : 7.77 (1H, s) ; 7.39-7.27 (6H, m) ; 5.01 (2H, s) ; 4.24-4.10 (3H, m) ; 3.35 (1H, br) ; 2.52 (3H, s) ; 1.97-1.80 (6H, m) ; 1.40 (2H, m) ; 1.26 (3H, t, J = 6.3 Hz).

### Etape 5: 1-(4-Benzyloxycarbonylamino-cyclohexyl)-5-methyl-1H-pyrazole-4-carboxylic acid (XV)

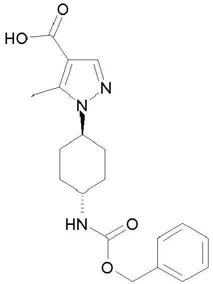

A 4.5 g (11.7 mmol) de 1-(4-Benzyloxycarbonylamino-*trans*-cyclohexyl)-5-methyl-1H-pyrazole-4-carboxylic acid ethyl ester préparé à l'étape précédente dans 20 mL d'un mélange THF/éthanol (1/1 ; v/v) est ajouté une solution de 3.27 g (58.4 mmol) d'hydroxyde de potassium dans l'eau. Le milieu réactionnel est chauffé à 50°C pour la nuit. Après refroidissement, de l'eau est ajouté et les insaponifiables sont extrait à l'éther iso. La phase aqueuse est acidifiée à pH 5 à l'aide d'une solution aqueuse d'acide chlorhydrique. Le précipité blanc formé est filtré, lavé à l'éther iso puis séché à l'étuve à vide à 60°C pour obtenir le produit attendu sous la forme d'une poudre beige.

Rendement = 90 %.

RMN ¹H : DMSO-d₆ (400 MHz) : δ (ppm) : 12.11 (1H, br) ; 7.73 (1H, s) ; 7.39-7.27 (6H, m) ; 5.01 (2H, s) ; 4.14 (1H, m) ; 3.33 (1H, br) ; 2.50 (3H, s) ; 1.98-1.81 (6H, m) ; 1.40 (2H, m).

### Etape 6: 3-[1-(4-Benzyloxycarbonylamino-cyclohexyl)-5-methyl-1H-pyrazol-4-yl]-3-oxo-propionic acid ethyl ester (XVI)

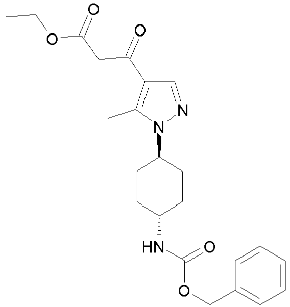

A 3.8 g (10.6 mmol) de 1-(4-Benzyloxycarbonylamino-cyclohexyl)-5-methyl-1H-pyrazole-4-carboxylic acid dans 110 ml de THF, sont ajoutés 1.97 g (15.9 mmol) de 1,1'-carbonyldiimidazole. Le milieu réactionnel est agité à TA pendant 2h. 4.6 g de magnésium bis (3-éthoxy-3-oxopropanoate) (synthétisé selon la méthode décrite dans Angew. Chem. Int. Ed. Engl., 1979, 18, 72-74) sont ajoutés et le milieu réactionnel est chauffé à 50°C pendant 3 heures. Le mélange réactionnel est évaporé sous vide et repris à l'AcOEt. La phase organique est lavée successivement avec une solution saturée de Na₂CO₃, à l'eau et à la saumure puis séchée sur Na₂SO₄. Le solvant est évaporé sous vide. Le résidu solide est trituré à l'éther iso, filtré puis séché à l'étuve à vide à 60°C pour obtenir 2.7 g d'une poudre rose.

### Rendement = 59 %.

RMN ¹H : DMSO-d₆ (400 MHz) : δ (ppm) : 8.02 (1H, s) ; 7.39-7.27 (6H, m) ; 5.01 (2H, s) ; 4.18 (1H, m) ; 4.09 (2H, q, J = 7 Hz) ; 3.87 (2H, s) ; 3.35 (1H, m) ; 2.53 (3H, s) ; 1.97-1.79 (6H, m) ; 1.41 (2H, m) ; 1.18 (3H, t, J = 7 Hz).

### Etape 7: 2-[1-(4-Benzyloxycarbonylamino-cyclohexyl)-5-methyl-1H-pyrazole-4-carbonyl]-butyric acid ethyl ester (VI)

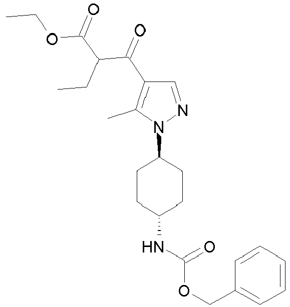

Dans un tube scellé sont placés 2.4 g (5.2 mmol) de 3-[1-(4-Benzyloxycarbonylamino-cyclohexyl)-5-methyl-1H-pyrazol-4-yl]-3-oxo-propionic acid ethyl ester préparé à l'étape précédente dans 40 mL de THF. 3.36 g (10.4 mmol) de bromure de tétrabutylammonium, 2.85 g (20.7 mmol) de K₂CO₃ et 3.75 mL (38.7 mmol) d'iodoéthane sont ajoutés et le milieu réactionnel est chauffé à 50°C pendant la nuit. Le milieu réactionnel est évaporé à sec puis repris à l'AcOEt. La phase organique est lavée à l'eau (1x), avec une solution de NaHCO₃ saturée (2x) et à la saumure (1x), puis séchée sur Na2SO₄ et évaporée sous vide. Le résidu solide est trituré à l'éther iso, filtré puis séché à l'étuve à vide à 60°C pour obtenir 3.1 g d'une poudre beige contenant le produit attendu et une mole d'une impureté correspondant à un dérivé de tétrabutylammonium. Le brut réactionnel est engagé dans l'étape suivante (Rendement approximatif en produit attendu = 82%).

RMN ¹H : DMSO-d₆ (400 MHz) : δ (ppm) : 8.09 (1H, s) ; 7.39-7.27 (6H, m) ; 5.01 (2H, s) ; 4.24-4.02 (4H, m) ; 3.35 (1H, m) ; 2.53 (3H, s) ; 1.97-1.72 (8H, m) ; 1.40 (2H, m) ; 1.12 (3H, t, J = 7 Hz) ; 0.87 (3H, t, J = 7 Hz).

### Etape 7: 1-[1-(4-Amino-cyclohexyl)-5-methyl-1H-pyrazol-4-yl]-butan-1-one

(V):R₁=nPr;R₂=Me;A=

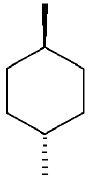

0.6 g du brut réactionnel obtenu à l'étape précédente (contenant - 0.37 g soit 0.81 mmol de 2-[1-(4-Benzyloxycarbonylamino-cyclohexyl)-5-methyl-1H-pyrazole-4-carbonyl]-butyric acid ethyl ester est chauffé à reflux dans HCl 6N pendant 4h. Après refroidissement, 10 mL d'une solution aqueuse de soude à 35% est ajouté (le pH final doit être basique). La phase aqueuse est extraite au DCM (3x). Les phases organiques rassemblées sont lavées successivement à l'eau (1x), à l'aide d'une solution de NaHCO₃ saturée (1x) et à la saumure puis séchées sur Na₂SO₄ et évaporées à sec. Le résidu solide est trituré à l'éther iso, filtré puis séché à l'étuve à vide à 60°C pour obtenir 0.6 g d'une poudre beige contenant le produit attendu et une mole d'une impureté correspondant à un dérivé de tétrabutylammonium (RMN difficile à déconvoluer). Le brut réactionnel est engagé tel quel dans l'étape suivante.

### Préparation 2.2

### 1-[1-(1-Amino-piperidin-4-yl)-5-methyl-1H-pyrazol-4-yl]-butan-1-one

(V):R₁=n-Pr; R₂ = Me; A=

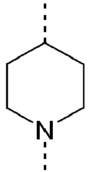

### Etape 1: 3-(tert-Butyl-dimethyl-silanyloxy)-pentanedioic acid dimethyl ester (XXIII)

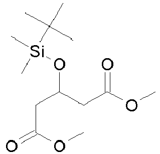

A une solution contenant 28.2 g (0.19 mole) de *tert*-butyl-chloro-dimethylsilane 30.2 g (0.44 mol) d'imidazole dans 225 ml de DCM, est ajouté lentement une solution contenant 30 g (0.17 mole) de dimethyl 3-hydroxyglutarate dans 225 ml de DCM. Après agitation pour la nuit, de l'eau est ajoutée, les phases sont séparées et la phase aqueuse est extraite au DCM (2x). Les phases organiques combinées sont séchées sur MgS04 puis evaporées à sec. Le résidu est purifié par chromatographie sur gel de silice en éluant avec un gradient acétate d'éthyle / heptane. On obtient 40.3 g du composé attendu sous forme de poudre.

Rendement = 82 %.

LC-MS tR(méthode A): 1.10 min; MS (ES) m/z: 291.3 (MH+).

RMN ¹H : DMSO-d₆ (500 MHz) : δ (ppm) : 0.00 (6H, s) ; 0.70 (9H, s) ; 3.62 (4H, 2 x d) ; 4.50 (1H, pent).

### Etape 2: 3-(tert-Butyl-dimethyl-silanyloxy)-pentane-1,5-diol (XXIV)

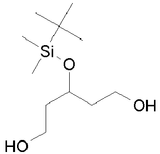

A une solution contenant 13.5 g (47 mmol) de 3-(*tert*-butyl-dimethyl-silanyloxy)-pentanedioic acid dimethyl ester dans 750 ml de diéthyléther à 0°C, sont ajoutés par portion 6.0 g (275 mmol) de borohydrure de lithium. Après agitation pour la nuit, une solution aqueuse saturée de chlorure d'ammonium est ajoutée. La phase aqueuse est extraite au DCM (2x) puis les phases organiques sont rassemblées et évaporées à sec pour donner 11.9 g de produit brut.

Rendement - quantitatif.

LC-MS tR(méthode A): 0.81 min; MS (ES) m/z: 235.3 (MH+).

RMN ¹H : DMSO-d₆ (500 MHz) : δ (ppm) : 0.00 (6H, s) ; 0.82 (9H, s) ; 1.52 (4H, m) ; 3.41 (4H, m) ; 3.86 (1H, pent) ; 4.28 (2H, t).

### Etape 3: [4-(tert-Butyl-dimethyl-silanyloxy)-piperidin-1-yl]-carbamic acid tert-butyl ester (XXV)

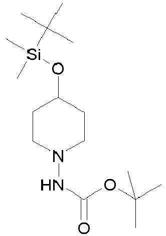

A une solution contenant 13.3 ml (155 mmol) de chlorure d'oxalyle dans 700 mL de DCM est ajouté goutte à goutte, à -78°C, une solution contenant 14.4 ml de diméthylsulfoxyde (203 mmol) dans 160 ml de DCM. Après 30 minutes d'agitation, une solution contenant 11.9 g (51 mmol) de 3-(tert-butyl-dimethyl-silanyloxy)-pentane-1,5-diol dans 160 ml DCM est ajouté à -78°C. Après 30 minutes d'agitation supplémentaire, 70.7 ml (51 mmol) de triéthylamine sont ajoutées. Le milieu réactionnel est agité à -78°C pendant 1h, puis la température est portée à 0 °C pour 30 minutes d'agitation supplémentaire. 92 ml de toluène sont ajoutés, puis le mélange est filtré. Le filtrat est concentré puis resuspendu dans le pentane et filtré sur Celite® pour donner 13.5 g de produit brut après évaporation du solvant.

12 g de du brut réactionnel obtenu précédemment sont mélangés avec 7.6 g (58 mmol) de tert-butyl carbazate dans 350 ml de DCM. 26.5 g (125 mmol) de triacétoxyborohydrure de sodium sont ajoutés à 0 °C puis le milieu réactionnel est agité pour la nuit. Une solution aqueuse saturée de NaHCO₃ est ajouté et les phases sont séparées. La phase aqueuse est extraite au DCM (2x) puis les phases organiques rassemblées sont évaporées à sec. Le résidu est purifié par chromatographie sur gel de silice en éluant avec un gradient acétate d'éthyle / heptane pour obtenir 8.6 g de produit attendu.

Rendement = 51%.

LC-MS tR(méthode A): 1.03 min; MS (ES) m/z: 331.3 (MH+).

### Etape 4: (4-Hydroxy-piperidin-1-yl)-carbamic acid tert-butyl ester (XXVI)

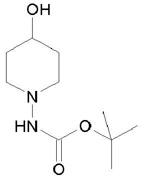

A une solution contenant 8.6 g (26 mmol) de [4-(tert-butyl-dimethyl-silanyloxy)-piperidin-1-yl]-carbamic acid tert-butyl ester dans 400 ml de THF, sont ajoutés 28.6 ml (28.6 mmol) de TBAF (1 M in THF) à 0°C. Après 12 h d'agitation, 28.6 mL de TBAF sont ajoutés et le milieu réactionnel est agité pour 12 h supplémentaires. Après concentration à sec, le résidu est redissout dans le DCM et extrait à l'eau (3x). Les phases aqueuses combinées sont réextraites à l'aide d'un mélange DCM/i-PrOH (3:1) et les phases organiques combinées sont évaporées à sec. Le résidu est purifié par chromatographie sur gel de silice en éluant avec un gradient acétate d'éthyle / heptane pour obtenir 4.4 g de produit attendu.

Rendement = 78 %.

LC-MS tR(méthode A): 0.25 min; MS (ES) m/z: 217.3 (MH+).

RMN ¹H : DMSO-d₆ (500 MHz) : δ (ppm) : 1.37 (9H, s) ; 1.43 (2H, m) ; 1.69 (2H, m) ; 2.53 (2H, m) ; 2.82 (2H, m) ; 3.47 (1H, m) ; 4.62 (1H, d) ; 7.97 (1H, d).

### Etape 5: Methanesulfonic acid 1-tert-butoxycarbonylamino-piperidin-4-yl ester

(X : Y = -OSO₂Me ; A = 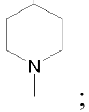 Pg = *tert*-butoxycarbonyle)

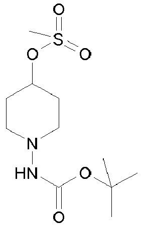

A une solution contenant 4.3 g (20 mmol) de (4-hydroxy-piperidin-1-yl)-carbamic acid tert-butyl ester dans 100 ml de DCM, sont ajoutés 0.24 g (2.0 mmol) de DMAP, 2.8 mL (20 mmol) de triéthylamine and 1.5 mL (20 mmol) de chlorure de methanesulfonyle. Après agitation pour la nuit, le milieu réactionnel est dilué au DCM et lavé à l'aide d'une solution aqueuse de HCl 0.1 M. La phase aqueuse est extraite au DCM et les phases organiques sont rassemblées pour donner 6.0 g d'une huile incolore.

Rendement = quantitatif.

LC-MS tR(méthode A): 0.61 min; MS (ES) m/z: 239.2 (M-tBu+H+).

RMN ¹H : DMSO-d₆ (500 MHz) : δ (ppm) : 1.40 (9H, s) ; 1.83 (2H, m) ; 1.95 (2H, m) ; 2.78 (2H, m) ; 2.92 (2H, m) ; 3.24 (3H, s) ; 4.76 (1H, m) ; 8.39 (1H, d).

### Etape 6: [4-(4-Butyryl-5-methyl-pyrazol-1-yl)-piperidin-1-yl]-carbamic acid tert-butyl ester

(VI:R1 = nPr; R2 = Me; A = 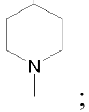 Pg = *tert*-butoxycarbonyle)

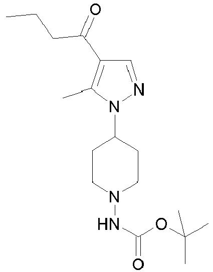

A une solution contenant 1.55 g (10 mmol) de 1-(5-methyl-1H-pyrazol-4-yl)-butan-1-one dans 15 mL de DMF, sont ajoutés 1.20 g (11 mmol) de *tert*-butylate de potassium et le mélange réactionnel est agité à 50°C pendant 30 minutes. Une solution contenant 3.00 g (10 mmol) de methanesulfonic acid 1-tert-butoxycarbonylamino-piperidin-4-yl ester dans 5 ml de DMF est ajoutée. Afin d'augmenter la conversion, 0.9 g de *tert-*butylate de potassium est ajouté à raison de 0.3 g toutes les 12 h. Le milieu est concentré, dilué au DCM et extrait à l'eau (3x). Le brut ainsi obtenu est purifié par CLHP préparative (colonne C18 phase inverse avec élution à l'aide d'un gradient eau/MeCN en présence de 0.1% de TFA) pour obtenir, après lyophilisation, 960 mg d'un mélange contenant le produit attendu et son isomère de position. Une purification supplémentaire par CLHP sur phase stationnaire chirale a permis d'isoler 360 mg de produit attendu.

Rendement = 10%.

LC-MS tR(méthode A): 0.82 min; MS (ES) m/z: 351.2 (MH+).

RMN ¹H : DMSO-d₆ (500 MHz) : δ (ppm) : 0.87 (3H, t) ; 1.39 (9H, s) ; 1.57 (2H, m) ; 1.82 (2H, d br) ; 2.13 (2H, m) ; 2.54 (3H, s) ; 2.73 (2H, t) ; 2.83 (2H, t br) ; 3.08 (2H, d br) ; 4.26 (1H, m) ; 8.14 (1H, s) ; 8.61 (1H, s br).

### Etape 7: Chlorhydrate du 1-[1-(1-Amino-piperidin-4-yl)-5-methyl-1H-pyrazol-4-yl]-butan-1-one

(V:R₁ = n-Pr; R₂ = Me; A =

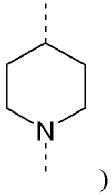

A une solution contenant 536 mg (1.53 mmol) de [4-(4-butyryl-5-methyl-pyrazol-1-yl)-piperidin-1-yl]-carbamic acid tert-butyl ester dans 12 ml de DCM sont ajoutés 3.3 mL d'acide trifluoroacetique. Après agitation pendant 3h, le milieu réactionnel est concentré puis codistillé deux fois avec du toluène. Le résidu ainsi obtenu est dissout dans un mélange MeCN/eau (1:1) puis lyophilisé après addition de 2.0 mL (4 mmol) de HCL 2N. Cette procédure est repétée une fois pour donner 350 mg de produit attendu sous la forme d'un chlorhydrate.

Rendement = 80 %.

LC-MS tR(méthode A): 0.47 min; MS (ES) m/z: 251.3 (MH+).

RMN ¹H : DMSO-d₆ (500 MHz) : δ (ppm) : 0.87 (3H, t), 1.55 (2H, m), 1.94 (2H, d br), 2.15 (2H, m) ; 2.55 (3H, s) ; 2.72 (2H, t) ; 2.90 (2H, s br) ; 3.33 (2H, d br) ; 4.43 (1H, m) ; 8.10 (1H, s).

### 3. Préparations des composés de formule (IV).

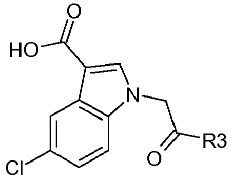

### Préparation 3.1

### Acide 5-chloro-1-(2-méthoxy-2-oxoéthyl)-1H-indole-3-carboxylique.

(IV-A) : R3 = OMe ; R₄ = Cl

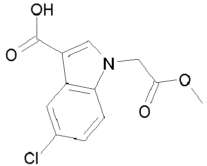

A un mélange de 4,50 g (112 mmol) de NaH (60% dans l'huile) dans 400 ml de DMF à -10°C, sont ajoutés, goutte à goutte, 110 ml d'une solution contenant 10 g (51 mmol) d'acide 5-chloro-1*H*-indole-3-carboxylique (commercial) dans le DMF et laisse sous agitation pendant 1h. Le mélange réactionnel est refroidit à -20°C puis 4,86 ml (51 mmol) de bromoacétate de méthyle sont ajoutés goutte à goutte. La remontée à TA est effectuée sur une période de 5 heures puis le milieu réactionnel est agité à TA pendant 15h. Le milieu réactionnel est ajouté à 1L d'un mélange AcOEt/HCl 1N, la phase organique est collectée et la phase aqueuse est extraite à l'AcOEt. Les phases organiques sont rassemblées, lavées à l'eau, à la saumure puis sèchées sur Na₂SO₄ et évaporées à sec pour obtenir 8,9 g du composé attendu sous forme d'une poudre blanche.

Rendement = 65%

RMN ¹H : DMSO-d₆ (250 MHz) : δ (ppm) : 3,70 (3H, s) ; 7,26 (1H, d) ; 7,57 (1H, d) ; 7,98 (1H,s) ; 8,12 (1H, s) ; 12,3 (1H, br).

### Préparation 3.2

5-Chloro-1-[2-(4-methyl-piperazin-1-yl)-2-oxo-ethyl]-1H-indole-3-carboxylic acid (IV-B) : R3 = 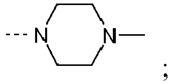 R₄ = Cl

### Etape 1: 5-Chloro-1H-indole-3-carboxylic acid tert-butyl ester (XXVIII)

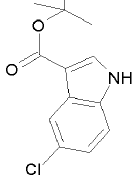

A une solution contenant 5.0 g (26 mmol) de 5-chloro-1H-indole-3-carboxylic acid dans 200 mL de benzène, sont ajoutés 25 mL (104 mmol) N,N-dimethylformamide di-tert-butyl acetal. Le milieu réactionnel est agité pendant 12 h, puis une autre portion de l'acétale (25 ml) est ajouté. Après 12h supplémentaires d'agitation, le milieu est concentré, dilué au DCM puis lavé avec une solution saturée de NaHCO₃ (3x). 5.7 g de produit attendu sont obtenus après évaporation des solvants.

Rendement = 87 %.

LC-MS tR(méthode A): 1.05 min; MS (ES) m/z: 196.1 (M-tBu+H+).

### Etape 2: 1-Benzyloxycarbonylmethyl-5-chloro-1H-indole-3-carboxylic acid tert-butyl ester (XXIX)

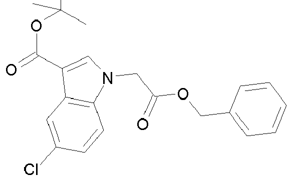

A une solution contenant 5.7 g (22 mmol) de 5-chloro-1H-indole-3-carboxylic acid tert-butyl ester et 3.5 mL (22 mmol) de bromoacetate de benzyle dans 150 ml de DMF, sont ajoutés 11.1 g (34 mmol) de carbonate de césium. Après 12h d'agitation, le milieu réactionnel est dilué au DCM puis lavé 3x avec une solution aqueuse de LiCl (4 % w/w). La phase organique est séchée sur MgSO₄ et le résidu obtenu après évaporation est purifié par chromatographie sur gel de silice en éluant avec un gradient acétate d'éthyle / méthanol.pour obtenir 5.8 g de produit attendu.

Rendement = 66 %

LC-MS tR(méthode A): 1.22 min; MS (ES) m/z: 344.2 (M-tBu+H+).

### Etape 3: 1-Carboxymethyl-5-chloro-1H-indole-3-carboxylic acid tert-butyl ester (XXX)

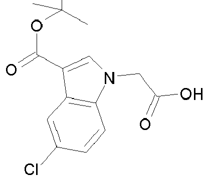

A une solution contenant 9.8 g (24 mmol) de 5-chloro-1H-indole-3-carboxylic acid tert-butyl ester dans un mélange 140 mL d'un THF/Eau (4:1), sont ajoutés 12.2 mL (24 mmol) d'une solution de LiOH 2M à 0°C. Après 3h d'agitation, le pH du milieu réactionnel est ajusté à 6 par addition de HCl 1N. Lors de l'évaporation des solvants, un solide se forme qui est collecté par filtration pour donner 4.3 g d'une poudre blanche. L'extraction du filtrat a permis l'obtention de 4 g supplémentaires de produit attendu.

Rendement = quantitatif.

LC-MS tR(méthode A): 0.96 min; MS (ES) m/z: 254.2 (M-tBu+H+).

### Etape 4: 5-Chloro-1-[2-(4-methyl-piperazin-1-yl)-2-oxo-ethyl]-1H-indole-3-carboxylic acid tert-butyl ester

(XXXI : R3 = 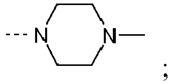 R₄=Cl)

A une solution contenant 3.15 g (10 mmol) of 1-carboxymethyl-5-chloro-1H-indole-3-carboxylic acid tert-butyl ester dans 70 ml de DMF, sont ajoutés 2.93 g (20 mmol) d'EDC et 2.81 g (15 mmol) de pentafluorophenol. Après 20 minutes d'agitation, 3.8 mL (30 mmol) de N-éthylmorpholine et 1.1 mL (10 mmol) de 1-methyl-piperazine sont ajoutés et le milieu réactionnel est agité pendant 12h à TA. Après évaporation du solvant, le résidu est repris au DCM et lavé 2x avec une solution aqueuse de LiCl (4 % w/w). La phase organique est séchée sur MgSO₄, évaporée à sec puis le produit brut obtenu est purifié par chromatographie sur gel de silice en éluant avec un gradient acétate d'éthyle / méthanol pour obtenir 2.26 g de produit attendu.

Rendement = 58 %.

LC-MS tR(méthode A): 0.73 min; MS (ES) m/z: 336.2 (M-tBu+H+).

RMN ¹H : DMSO-d₆ (500 MHz) : δ (ppm) : 1.58 (9H, s) ; 2.24 (3H, s) ; 2.31 (2H, m) ; 2.44 (2H, m) ; 3.47 (2H, m) ; 3.58 (2H, m) ; 5.34 (2H, s) ; 7.28 (1H, dd) ; 7.54 (1H, d);7.99(1H,d);8.05(1H,s).

### Etape 5: 5-Chloro-1-[2-(4-methyl-piperazin-1-yl)-2-oxo-ethyl]-1H-indole-3-carboxylic acid

(IV-B : R3 = 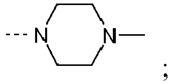 R₄=Cl)

A une solution contenant 2.26 g (5.8 mmol) de 5-Chloro-1-[2-(4-methyl-piperazin-1-yl)-2-oxo-ethyl]-1H-indole-3-carboxylic acid tert-butyl ester dans 30 ml de DCM, sont ajoutés 4.3 mL d'acide trifluoroacetique. Après agitation pendant 3h, le milieu réactionnel est concentré puis codistillé avec du toluène (2x). Le résidu ainsi obtenu est dissout dans un mélange MeCN / eau et lyophilisé après addition de 7.3 mL de HCl 2N. Cette procédure est répétée une fois pour obtenir 2.33 g du chlorhydrate du produit attendu.

Rendement = quantitatif.

LC-MS tR(méthode A): 0.51 min; MS (ES) m/z: 336.2 (MH+).

RMN ¹H : DMSO-d₆ (500 MHz) : δ (ppm) : 2.79 (3H, s) ; 2.96 (1H, m) ; 3.13 (2H, m) ; 3.43 (1H, d) ; 3.52 (1H, d) ; 3.62 (1H, t) ; 4.15 (1H, d) ; 4.37 (1H, d) ; 5.34 (1H, d) ; 5.50 (1H, d) ; 7.31 (1H, dd) ; 7.61 (1H, d) ; 8.04 (1H, d) ; 8.06 (1H, s) ; 11.25 (1H, s br).

### Préparation 3.3

### 5-Chloro-1-{2-[4-(2-methoxy-ethyl)-piperazin-1-yl]-2-oxo-ethyl}-1H-indole-3-carboxylic acid

(IV-B) : R3 = 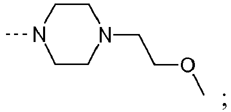 R₄=Cl

### Etape 1: 5-Chloro-1-{2-[4-(2-methoxy-ethyl)-piperazin-1-yl]-2-oxo-ethyl}-1H-indole-3-carboxylic acid tert-butyl ester

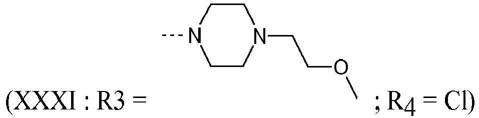

A une solution contenant 4.3 g (14 mmol) of 1-carboxymethyl-5-chloro-1H-indole-3-carboxylic acid tert-butyl ester dans 95 ml de DMF, sont ajoutés 3.99 g (28 mmol) d'EDC et 3.83 g (21 mmol) de pentafluorophenol. Après 20 minutes d'agitation, 5.5 mL (42 mmol) de N-éthylmorpholine et 2.1 mL (14 mmol) de 1-(2-methoxyéthyl)piperazine sont ajoutés et le milieu réactionnel est agité pendant 12h à TA. Après évaporation du solvant, le résidu est repris au DCM et lavé 2x avec une solution aqueuse de LiCl (4 % w/w). La phase organique est séchée sur MgSO₄, évaporée à sec puis le produit brut obtenu est purifié par chromatographie sur gel de silice en éluant avec un gradient acétate d'éthyle / méthanol pour obtenir 5.4 g de produit attendu.

Rendement = 87 %.

LC-MS tR(méthode A): 0.75 min; MS (ES) m/z: 436.4 (MH+).

### Etape 2: 5-Chloro-1- {2-[4-(2-methoxy-ethyl)-piperazin-1-yl]-2-oxo-ethyl}-1H-indole-3-carboxylic acid

(IV-B : R3 = 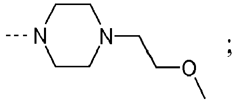 R₄=Cl)

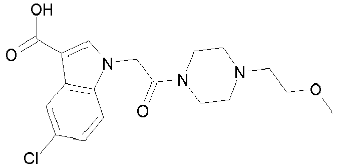

A une solution contenant 2 g (4.6 mmol) de 5-chloro-1-{2-[4-(2-methoxy-ethyl)-piperazin-1-yl]-2-oxo-ethyl}-1H-indole-3-carboxylic acid tert-butyl ester dans 25 ml de DCM, sont ajoutés 3.4 ml d'acide trifluoroacetique. Après agitation pendant 3h, le milieu réactionnel est concentré puis codistillé avec du toluène (2x). Le résidu ainsi obtenu est dissout dans un mélange MeCN / eau et lyophilisé après addition de 6.5 mL de HCl 1 N. Cette procédure est répétée une fois pour obtenir 1.92 g du chlorhydrate du produit attendu.

Rendement = quantitatif.

LC-MS tR(méthode A): 0.55 min; MS (ES) m/z: 380.3 (MH+).

### Préparation 3.4

### 5-Chloro-1-{2-[4-(2-methoxy-ethyl)-piperazin-1-yl]-2-oxo-ethyl}-1H-indole-3-carboxylic acid

(IV-B) : R3 = 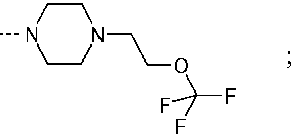 R₄=Cl

### Etape 1: 1-Benzyl-4-(2-trifluoromethoxy-ethyl)-piperazine

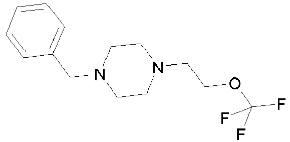

Une suspension de 0.77 g (4.7 mmol) de chlorhydrate de 2-(trifluorométhoxy)éthylamine et 1.8 g (21 mmol) de NaHCO₃ dans 100 mL d'éthanol sont agités à 80°C pendant 6h. Le milieu réactionnel est filtré, concentré. Le résidu est repris au DCM, lavé à l'eau (1x), puis la phase aqueuse est réextraite au DCM. Les phases organiques rassemblées sont évaporées puis le produit brut est purifié par chromatographie sur gel de silice en éluant avec un gradient acétate d'éthyle / heptane pour obtenir 419 mg de produit attendu sous la forme d'une huile incolore.

Rendement =31%.

LC-MS tR(method A): 0.46 min; MS (ES) m/z: 289.3 (MH+).

RMN ¹H : DMSO-d₆ (500 MHz) : δ (ppm) : 2.33 - 2.52 (8H, m) ; 2.65 (2H, t) ; 3.52 (2H, s) ; 4.21 (2H, t) ; 7.31 - 7.41 (5H, m).

### Etape 2: 1-(2-Trifluoromethoxy-ethyl)-piperazine

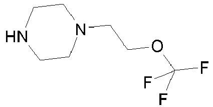

Une solution de 2.20 g (7.6 mmol) de 1-benzyl-4-(2-trifluoromethoxy-ethyl)-piperazine dans 50 ml de THF est transférée dans un autoclave Büchi puis hydrogénée pendant 9h à 3 bars en présence de 200 mg (0.29 mmol) de Pd(OH)2 à 20% sur charbon. La conversion n'étant pas complète, la suspension est filtré, du catalyseur frais est ajouté et le milieu réactionnel hydrogené pour 3 h supplémentaires. Après filtration sur Célite®, lavage à l'éthanol et évaporation à sec, 973 mg de produit débenzylé sont obtenus sous la forme d'une huile incolore.

### Rendement = 65 %.

LC-MS tR(méthode A): 0.09 min; MS (ES) m/z: 199.2 (MH+).

RMN ¹H : CDCl3 (400 MHz) : δ (ppm) : 2.53 - 2.65 (4H, m) ; 2.75 (2H, t) ; 3.02 (4H, m) ; 3.59 (1H, m) ; 4.15 (2H, t).

### Etape 3: 5-Chloro-1-{2-oxo-2-[4-(2-trifluoromethoxy-ethyl)-piperazin-1-yl]-ethyl}-1H-indole-3-carboxylic acid tert-butyl ester

(XXXI) : R3 = 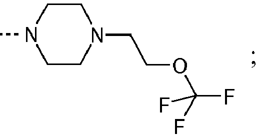 R₄=Cl

A une solution contenant 600 mg (1.9 mmol) de 1-carboxymethyl-5-chloro-1H-indole-3-carboxylic acid tert-butyl ester dans 30 ml de DMF, sont ajoutés 557 mg (3.8 mmol) d'EDC et 534 mg (2.9 mmol) de pentafluorophenol. Après 20 minutes d'agitation, 0.7 mL (5.7 mmol) de N-éthylmorpholine et 416 mg (2.0 mmol) de 1-(2-Trifluoromethoxy-ethyl)-piperazine sont ajoutés et le milieu réactionnel est agité pendant 12h à TA. Après évaporation du solvant, le résidu est repris au DCM et lavé 2x avec une solution aqueuse de LiCl (4 % w/w). La phase organique est séchée sur MgSO₄, évaporée à sec puis le produit brut obtenu est purifié par chromatographie sur gel de silice en éluant avec un gradient acétate d'éthyle / heptane pour obtenir 634 mg de produit attendu.

Rendement = 68 %.

LC-MS tR(méthode A): 0.75 min; MS (ES) m/z: 436.4 (MH+).

### Etape 4: 5-Chloro-1- {2-oxo-2-[4-(2-trifluoromethoxy-ethyl)-piperazin-1-yl]-ethyl}-1H-indole-3-carboxylic acid

(IV-B) : R3 = 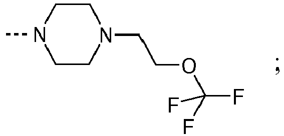 R₄=Cl

A une solution contenant 634 mg (1.4 mmol) de 5-chloro-1-(2-[4-(2-methoxy-ethyl)-piperazin-1-yl]-2-oxo-ethyl}-1H-indole-3-carboxylic acid tert-butyl ester dans 10 ml de DCM, sont ajoutés 0.9 ml d'acide trifluoroacetique. Après agitation pendant 3h, le milieu réactionnel est concentré puis codistillé avec du toluène (2x). Le résidu ainsi obtenu est dissout dans un mélange MeCN / eau et lyophilisé après addition de 1.3 mL de HCl 2N. Cette procédure est répétée une fois pour obtenir 567 mg du chlorhydrate du produit attendu.

Rendement = 94 %.

LC-MS tR(method A): 0.55 min; MS (ES) m/z: 380.3 (MH+).

### 4 Préparation du composé {3-[4-(4-Butyryl-5-methyl-pyrazol-1-yl)-trans-cyclohexylcarbamoyl]-5-chloro-indol-1-yl}-acetic acid

(IIB : R1 = nPr;R2 = Me;A = 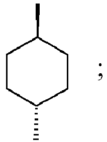 R3 = OH ; R4 = Cl)

### Etape 1 : {3-[4-(4-Butyryl-5-methyl-pyrazol-1-yl)-trans-cyclohexylcarbamoyl]-5-chloro-indol-1-yl}-acetic acid methyl ester

(IIA : R1 = nPr ; R2 = Me ; A = 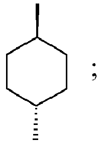 R3 = OZ ; R4 = Cl ; Z = Me)

A une solution contenant 600 mg (2.0 mmol) de 5-Chloro-1-methoxycarbonylmethyl-1H-indole-3-carboxylic acid (préparation 2.1), 528 mg (4.3 mmol) de DMAP dans 12 mL de DCM, sont ajoutés 439 mg (2.2 mmol) d'EDC. Après quelques minutes d'agitation, le milieu devient homogène et 610 mg (2.45 mmol) de 1-[1-(4-Amino-*trans*-cyclohexyl)-5-methyl-1H-pyrazol-4-yl]-butan-1-one (préparation 1.1) sont ajoutés. Après agitation à TA pendant 48 h, le milieu réactionnel est lavé à l'eau (1x), à l'HCl 1N (1x), avec NaHCO₃ (1x) puis à la saumure. La phase organique est séchée sur Na₂SO₄ puis évaporée à sec pour obtenir 1.1 g d'huile brune. Le résidu huileux est purifié par chromatographie sur gel de silice en éluant avec un gradient DCM / méthanol. L'huile jaune obtenue (550 mg).est reprise à l'acétone puis versée goutte à goutte sur éther iso. Le précipité blanc formé est filtré, lavé à l'éther iso et séché sous vide pour obtenir 300 mg d'une poudre blanche.

Rendement = 30 %.

RMN ¹H : DMSO-d₆ (400 MHz) : δ (ppm) : 8.15 (1H, d, J = 2.2 Hz) ; 8.08 (1H, s) ; 8.03 (1H, s) ; 7.93 (1H, d, J = 7.7 Hz) ; 7.52 (1H, d, J = 8.9 Hz) ; 7.21 (1H, dd, J = 8.9, 2.2 Hz) ; 5.25 (2H, s) ; 4.22 (1H, m) ; 3.85 (1H, m) ; 3.70 (3H, s) ; 2.73 (2H, t, J = 7.2 Hz) ; 2.56 (3H, s) ; 2.05 - 1.85 (6H, m) ; 1.59 (2H, sext, J = 7.2 Hz) ; 1.55 (2H, m) ; 0.91 (3H, t, J = 7.2 Hz).

### Etape 2 : {3-[4-(4-Butyryl-5-methyl-pyrazol-1-yl)-trans-cyclohexylcarbamoyl]-5-chloro-indol-1-yl}-acetic acid

(IIB : R1 = nPr ; R2 = Me ; A = 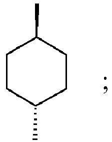 R3 = OH; R4 = Cl)

A une solution contenant 300 mg (0.60 mmol) de {3-[4-(4-Butyryl-5-methyl-pyrazol-1-yl)-trans-cyclohexylcarbamoyl]-5-chloro-indol-1-yl}-acetic acid methyl ester (préparé à l'étape précédente) dans 3 mL de méthanol, sont ajoutés 26 mg (0.66 mmol) de NaOH. Le milieu réactionnel est agité pendant 2 h puis évaporé à sec. Le résidu est repris à l'eau puis à 0.7 mL d'HCl 1N sont ajoutés. Le précipité formé est filtré, lavé à l'eau puis séché à l'étuve à vide à 60°C pour obtenir 260 mg d'une poudre blanche.

Rendement = 89 %.

RMN ¹H : DMSO-d₆ (400 MHz) : δ (ppm) : 8.15 (1H, d, J = 2.2 Hz) ; 8.09 (1H, s) ; 8.03 (1H, s) ; 7.90 (1H, d, J = 7.8 Hz) ; 7.49 (1H, d, J = 8.8 Hz) ; 7.19 (1H, dd, J = 8.8, 2.2 Hz) ; 5.03 (2H, s) ; 4.22 (1H, m) ; 3.85 (1H, m) ; 2.72 (2H, t, J = 7.2 Hz) ; 2.56 (3H, s) ; 2.05 - 1.85 (6H, m) ; 1.59 (2H, sextuplet, J = 7.2 Hz) ; 1.55 (2H, m) ; 0.91 (3H, t, J = 7.2 Hz).

### EXEMPLES

### EXEMPLE 1 : Composé n° 1

Chlorhydrate du 5-Chloro-1-[2-(4-methyl-piperazin-1-yl)-2-oxo-ethyl]-1H-indole-3-carboxylic acid [4-(4-butyryl-5-methyl-pyrazol-1-yl)-cyclohexyl]-amide.

260 mg (0.54 mmol) de {3-[4-(4-Butyryl-5-methyl-pyrazol-1-yl)-*trans-*cyclohexylcarbamoyl]-5-chloro-indol-1-yl}-acetic acid (préparation 3), 136 mg (1.3 mmol) de N-méthylpipérazine et 211 mg (0.80 mmol) de BOP-Cl dans 5mL de DCM sont agités pendant la nuit. Le milieu réactionnel est évaporé à sec puis le résidu solide est trituré avec une solution de NaHCO₃ saturée. Le précipité blanc formé est filtré, lavé à l'eau, séché à l'étuve à vide à 60°C puis purifié par chromatographie sur gel de silice en éluant avec un gradient DCM / méthanol pour obtenir 290 mg de poudre blanche sous forme de base. Le produit est repris dans l'acétone puis 0.7 mL de HCl 1N dans le diéthyléther est ajouté. Le milieu est filtré et le précipité séché à l'étuve à vide à 60°C pour obtenir 260 mg d'une poudre blanche.

Rendement = 80 %.

LC-MS tR(méthode D): 6.1 min; MS (ES) m/z: 567 (MH+).

F = 214°C.

RMN ¹H : DMSO-d₆ (400 MHz) : δ (ppm) : 10.80 (1H, br) ; 8.15 (1H, d, J = 2.0 Hz) ; 8.03 (1H, s) ; 8.02 (1H, s) ; 7.92 (1H, d, J = 7.7 Hz) ; 7.49 (d, 1H, J = 8.9 Hz) ; 7.20 (1H, dd, J = 8.9, 2.0 Hz) ; 5.41 (1H, système AB: J = 17 Hz) ; 5.27 (1H, système AB: J = 17 Hz) ; 4.37 (1H, m) ; 4.20 (2H, m) ; 3.85 (1H, m) ; 3.56 (2H, m) ; 3.45 (1H, m) ; 3.12 (2H, m) ; 2.97 (1H, m) ; 2.83 (3H, s) ; 2.73 (2H, t, J = 7.2 Hz) ; 2.56 (3H, s) ; 2.05 - 1.85 (6H, m) ; 1.59 (2H, sext, J = 7.2 Hz) ; 1.55 (2H, m) ; 0.90 (3H, t, J = 7.2 Hz).

### EXEMPLE 2 : Composé n° 2

Chlorhydrate du 5-Chloro-1-{2-[4-(2-methoxy-ethyl)-piperazin-1-yl]-2-oxo-ethyl}-1H-indole-3-carboxylic acid [4-(4-butyryl-5-methyl-pyrazol-1-yl)-cyclohexyl]-amide.

250 mg (0.52 mmol) de {3-[4-(4-Butyryl-5-methyl-pyrazol-1-yl)-*trans-*cyclohexylcarbamoyl]-5-chloro-indol-1-yl}-acetic acid (préparation 3), 112 mg (0.77 mmol) de 1-(2-Methoxy-ethyl)-piperazine, 191 mg (1.5 mmol) de DMAP et 203 mg (0.77 mmol) de BOP-Cl dans 3.4 mL de DCM sont agités pendant la nuit. Le milieu réactionnel est évaporé à sec puis le résidu solide est trituré avec une solution de NaHCO₃ saturée. Le précipité est purifié par chromatographie sur gel de silice en éluant avec un gradient DCM / méthanol pour obtenir 240 mg de poudre blanche sous forme de base. Le produit est repris dans l'acétone puis 0.5 mL de HCl 1N dans le diéthyléther est ajouté. Le milieu est filtré et le précipité séché à l'étuve à vide à 60°C pour obtenir 200 mg d'une poudre blanche.

Rendement = 59 %.

LC-MS tR(méthode D): 6.2 min; MS (ES) m/z: 611 (MH+).

F = 263°C.

RMN ¹H : DMSO-d₆ (400 MHz) : δ (ppm) : 10.56 (1H, br) ; 8.15 (1H, d, J = 2.0 Hz) ; 8.03 (1H, s) ; 8.02 (1H, s) ; 7.91 (1H, d, J = 7.7 Hz) ; 7.49 (d, 1H, J = 8.9 Hz) ; 7.20 (1H, dd, J = 8.9, 2.0 Hz) ; 5.41 (1H, système AB: J = 17 Hz) ; 5.27 (1H, système AB: J = 17Hz) ; 4.35 (1H, m) ; 4.20 (2H, m) ; 3.86 (1H, m) ; 3.74 (2H, m) ; 3.70 - 3.47 (3H, m) ; 3.38 (2H, m) ; 3.34 (3H, s) ; 3.18 (2H, m) ; 3.02 (1H, m) ; 2.73 (2H, t, J = 7.2 Hz) ; 2.56 (3H, s) ; 2.05 - 1.85 (6H, m) ; 1.65-1.49 (4H, m) ; 0.90 (3H, t, J = 7.2 Hz).

### EXEMPLE 3 : Composé n° 3 Chlorhydrate du 5-Chloro-1-{2-oxo-2-[4-(3,3,3-trifluoro-propyl)-piperazin-1-yl]-ethyl}-1H-indole-3-carboxylic acid [4-(4-butyryl-5-methyl-pyrazol-1-yl)-cyclohexyl]-amide.

150 mg (0.31 mmol) de {3-[4-(4-Butyryl-5-methyl-pyrazol-1-yl)-*trans-*cyclohexylcarbamoyl]-5-chloro-indol-1-yl}-acetic acid (préparation 3), 118 mg (0.46 mmol) du dichlorhydrate de la 1-(3,3,3-Trifluoro-propyl)-piperazine, 229 mg (1.9 mmol) de DMAP et 122 mg (0.46 mmol) de BOP-Cl dans 2 mL de DCM sont agités pendant la nuit. Le milieu réactionnel est évaporé à sec puis le résidu solide est trituré avec une solution de NaHCO₃ saturée. Le précipité est purifié par chromatographie sur gel de silice en éluant avec un gradient DCM / méthanol pour obtenir 140 mg de poudre blanche sous forme de base. Le produit est repris dans le DCM puis 0.28 mL de HCl 1N dans le diéthyléther est ajouté. Le milieu est filtré et le précipité séché à l'étuve à vide à 60°C pour obtenir 115 mg d'une poudre blanche.

Rendement = 54 %.

LC-MS tR(méthode D): 6.98 min; MS (ES) m/z: 650 (MH+).

F = 249°C.

RMN ¹H : DMSO-d₆ (400 MHz) : δ (ppm) : 11.41 (1H, br) ; 8.15 (1H, d, J = 2.0 Hz) ; 8.03 (1H, s) ; 8.02 (1H, s) ; 7.90 (1H, d, J = 7.7 Hz) ; 7.48 (d, 1H, J = 8.9 Hz) ; 7.20 (1H, dd, J = 8.9, 2.0 Hz) ; 5.39 (1H, br) ; 5.30 (1H, br) ; 4.38 (1H, m) ; 4.21 (2H, m) ; 3.86 (1H, m) ; 3.65 (3H, m) ; 3.18 (2H, m) ; 2.98 (3H, m) ; 2.73 (2H, t, J = 7.2 Hz) ; 2.56 (3H, s) ; 2.05 - 1.85 (6H, m) ; 1.65 - 1.49 (4H, m) ; 0.90 (3H, t, J = 7.2 Hz).

### EXEMPLE 4 : Composé n° 4

### Chlorhydrate du. 5-Chloro-1-[2-(4-methyl-piperazin-1-yl)-2-oxo-ethyl]-1H-indole-3-carboxylic acid [4-(4-butyryl-5-methyl-pyrazol-1-yl)-piperidin-1-yl]-amide

A une solution contenant 337 mg (1.0 mmol) de 5-chloro-1-{2-[4-methyl-piperazin-1-yl]-2-oxo-ethylt-1H-indole-3-carboxylic acid (préparation 2.2) dans 45 mL de DMF, sont ajoutés 152 mg (1.1 mmol) d'HOBt, 0.6 mL (3.5 mmol) de DIPEA et 191 mg (1 mmol) d'EDC. Après 10 minutes d'agitation, 330 mg (1.1 mmol) du chlorhydrate de 1-[1-(1-amino-piperidin-4-yl)-5-methyl-1H-pyrazol-4-yl]-butan-1-one (préparation 1.2) sont ajoutés et le milieu réactionnel est agité pendant 24 h. 95 mg d'EDC, 75 mg d'HOBt et 0.3 mL de DIPEA sont ajoutés et après 24 h supplémentaires, la conversion est complète. Le milieu réactionnel est évaporé à sec, et le résidu est purifié par CLHP préparative (colonne phase inverse C18 avec élution à l'aide d'un gradient H2O/MeCN en présence de 0.1% de TFA). Le produit ainsi obtenu est dissout dans un mélange MeCN / eau et lyophilisé après addition de 0.43 mL de HCl 1N. Cette procédure est répétée une fois pour obtenir 186 mg du chlorhydrate du produit attendu.

Rendement = 33 %.

LC-MS tR(method C): 1.26 min; MS (ES) m/z: 568.2 (MH+).

RMN ¹H : DMSO-d₆ (500 MHz) : δ (ppm) : 0.88 (3H, t) ; 1.57 (2H, dt) ; 2.05 (2H, m) ; 2.42 (2H, m) ; 2.59 (3H, s) ; 2.75 (2H, t) ; 2.83 (3H, d) ; 3.00 (1H, m) ; 3.19 (2H, m) ; 3.45 -3.71 (6H, m) ; 4.22 (2H, d) ; 4.41 (2H, d) ; 4.57 (1H, s br) ; 5.43 (1H, d) ; 5.57 (1H, d) ; 7.36 (1H, d) ; 7.66 (1H, d) ; 8.18 (1H, s) ; 8.21 (1H, s) ; 8.32 (1H, s) ; 11.40(1H,sbr).

### EXEMPLE 5 : Composé n° 5

### Chlorhydrate du. 5-Chloro-1- {2-[4-(2-methoxy-ethyl)-piperazin-1-yl-yl]-2-oxo-ethyl}-1H-indole-3-carboxylic acid [4-(4-butyryl-5-methyl-pyrazol-1-yl)-piperidin-1-yl]-amide

A une solution contenant 436 mg (1.3 mmol) de 5-Chloro-1-{2-[4-(2-methoxy-ethyl)-piperazin-1-yl]-2-oxo-ethyl}-1H-indole-3-carboxylic acid (préparation 2.3) dans 60 mL de DMF, sont ajoutés 200 mg (1.5 mmol) d'HOBt, 1.3 mL (7.5 mmol) de DIPEA et 250 mg (1.3 mmol) d'EDC. Après 10 minutes d'agitation, 300 mg (1.2 mmol) du chlorhydrate de 1-[1-(1-amino-piperidin-4-yl)-5-methyl-1H-pyrazol-4-yl]-butan-1-one (préparation 1.2) sont ajoutés et le milieu réactionnel est agité pendant 24 h. 83 mg d'EDC, 67 mg d'HOBt et 0.4 mL de DIPEA sont ajoutés et après 24 h supplémentaires, la conversion est complète. Le milieu réactionnel est évaporé à sec, et le résidu est dissout dans 250 mL d'un mélange DCM / iPrOH (3 1) puis lavé avec une solution aqueuse de LiCl (4% w/w). La phase organique est séchée sur MgSO₄, et le résidu est purifié par CLHP préparative (colonne phase inverse C18 avec élution à l'aide d'un gradient H₂O/MeCN en présence de 0.1% de TFA). Le produit ainsi obtenu est dissout dans un mélange MeCN / eau et lyophilisé après addition de 1 mL de HCl 1N. Cette procédure est répétée une fois pour obtenir 250 mg du chlorhydrate du produit attendu.

Rendement = 30 %.

LC-MS t_{R}(méthode B): 3.24 min; MS (ES) m/z: 612.3 (MH+).

RMN ¹H : DMSO-d₆ (500 MHz) : δ (ppm) : 0.88 (3H, t) ; 1.57 (2H, dt) ; 2.06 (2H, m) ; 2.43 (2H, m) ; 2.59 (3H, s) ; 2.74 (2H, t) ; 3.06 (1H, m) ; 3.24 (2H, m) ; 3.35 (3H, s) ; 3.39 (2H, d) ; 3.50 - 3.81 (8H, m) ; 4.21 (2H, d) ; 4.39 (2H, d) ; 4.59 (1H, s br) ; 5.43 (1H, d) ; 5.57 (1H, d) ; 7.36 (1H, d) ; 7.67 (1H, d) ; 8.18 (1H, s) ; 8.21 (1H, s) ; 8.35 (1H, s) ; 11.21 (1H, s br).

### EXEMPLE 6 : Composé n° 6

### Chlorhydrate du. 5-Chloro-1- f 2-oxo-2-[4-(2-trifluoromethoxy-ethyl)-piperazin-1-yl]-ethyl}-1H-indole-3-carboxylic acid [4-(4-butyryl-5-methyl-pyrazol-1-yl)-piperidin-1-yl]-amide

A une solution contenant 492 mg (1.3 mmol) de 5-Chloro-1-{2-[4-(2-methoxy-ethyl)-piperazin-1-yl]-2-oxo-ethylt-IH-indole-3-carboxylic acid (préparation 2.4) dans 60 mL de DMF, sont ajoutés 200 mg (1.5 mmol) d'HOBt, 1.3 mL (7.5 mmol) de DIPEA et 250 mg (1.3 mmol) d'EDC. Après 10 minutes d'agitation, 300 mg (1.2 mmol) du chlorhydrate de 1-[1-(1-amino-piperidin-4-yl)-5-methyl-1H-pyrazol-4-yl]-butan-1-one (préparation 1.2) sont ajoutés et le milieu réactionnel est agité pendant 24 h. 83 mg d'EDC, 67 mg d'HOBt et 0.4 mL de DIPEA sont ajoutés et après 24 h supplémentaires, la conversion est complète. Le milieu réactionnel est évaporé à sec, et le résidu est dissout dans 250 mL d'un mélange DCM / iPrOH (3 :1) puis lavé avec une solution aqueuse de LiCl (4% w/w). La phase organique est séchée sur MgS04, et le résidu est purifié par CLHP préparative (colonne phase inverse C18 avec élution à l'aide d'un gradient MeCN / eau en présence de 0.1% de TFA). Le produit ainsi obtenu est dissout dans un mélange MeCN / eau et lyophilisé après addition de 1 mL de HCl 1N. Cette procédure est répétée une fois pour obtenir 250 mg du chlorhydrate du produit attendu.

Rendement = 29 %.

LC-MS tR(méthode B): 3.62 min; MS (ES) m/z: 666.3 (MH+).

RMN ¹H : DMSO-d₆ (500 MHz) : δ (ppm) : 0.88 (3H, t) ; 1.57 (2H, dt) ; 1.99 (2H, m) ; 2.34 (2H, m) ; 2.58 (3H, s) ; 2.74 (2H, t) ; 3.04 - 3.67 (12H, m) ; 4.25 (1H, d) ; 4.47 (2H, d) ; 4.67 (2H, s br) 5.42 (1H, s br) 5.53 (1H, s ber) 7.32 (1H, d) ; 7.62 (1H, d) ; 8.16 (1H, s); 8.19 (2H, s) ; 11.52 (1H, s br).

Les composés selon l'invention ont fait l'objet d'essais pharmacologiques.

### Inhibition de l'agrégation plaquettaire in vitro (sang rat)

Le sang est prélevé sur des rats mâles de souche Sprague-Dawley, pesant 250-300g. Le prélèvement sur citrate de sodium à 3.8% (1 volume pour 9 volumes de sang) est effectué par ponction à l'aorte abdominale après anesthésie de l'animal au pentobarbital sodique.

Le plasma riche en plaquettes (PRP) est obtenu par centrifugation du sang à 300g pendant 5 minutes, et les mesures d'agrégation plaquettaire sont réalisées comme décrites ci-dessus.

Les résultats sont calculés en utilisant l'aire sous la courbe d'absorbance mesurée pendant 6 minutes, et exprimés par le pourcentage d'inhibition.

Les composés selon l'invention présentent des C_{I50} (d'inhibition de l'agrégation plaquettaire) comprises entre 0,02 et 1.5 µM.

Les résultats obtenus pour chaque composé sont indiqués dans le tableau I.

**TABLEAU I**

| Composés n° | Formule développée | Inhibition de l'agrégation plaquettaire *in vitro* (sang rat) CI₅₀ en µM |
|---|---|---|
| 1 | 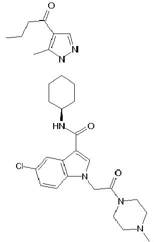 | 0.091 |
| 2 | 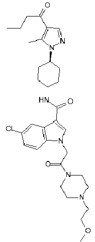 | 0.080 |
| 3 | 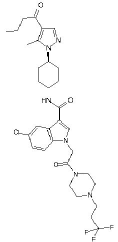 | 0.294 |
| 4 | 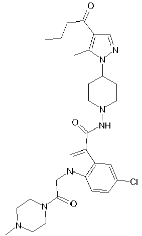 | 0.043 |
| 5 | 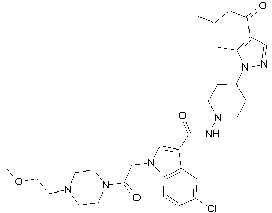 | 0.017 |
| 6 | 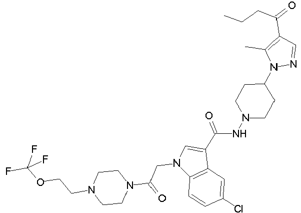 | 0.046 |

### Inhibition de l'agrégation plaquettaire in vitro (sang humain)

Le sang est prélevé chez des volontaires sains, en utilisant des seringues de 20ml contenant 2ml de citrate de sodium tamponné. Le sang est transféré dans des tubes en polypropylène, et centrifugé pendant 5 minutes (100g) à température ambiante (sans utiliser le frein de la centrifugeuse). Le plasma riche en plaquettes (PRP) surnageant est alors prélevé, dilué, et numéré en plaquettes avant d'être utilisé dans les mesures d'agrégation.

Les mesures d'agrégation plaquettaire sont réalisées à 37°C dans des tubes en verre (agrégomètre Chrono-Log - Kordia). 4µl du composé à tester (solution 100 fois plus concentrée que la concentration finale désirée, en DMSO) sont mélangés à 392µl de PRP frais, et incubés pendant 1 minute sous agitation. Puis, 4µl d'une solution d'ADP à 250µM sont ajoutés au mélange. Les mesures d'agrégation sont suivies pendant 6 à 8 minutes, sous agitation permanente, par enregistrement des variations de la densité optique selon la méthode de G.V.R. BORN (Born Nature (1962) 194, 927).

Les résultats sont calculés en utilisant l'amplitude de l'agrégation exprimée en hauteur, et exprimés par le pourcentage d'inhibition.

Les composés selon l'invention présentent des C1₅₀ (d'inhibition de l'agrégation plaquettaire) comprises entre 0,1 et 2 µM.

Le tableau II ci-après indique les résultats obtenus pour les composés 1 et 4 :

**TABLEAU II**

| Composés n° | Inhibition de l'agrégation plaquettaire *in vitro* (sang humain) CI₅₀ en µM |
|---|---|
| 1 | 0.27 |
| 4 | 0.025 |

### Inhibition de l'agrégation plaquettaire ex vivo (sang rat)

Des rats mâles de souche Sprague-Dawley, pesant 250-300g, sont utilisés à raison de 6 animaux par lot. Chaque composé à tester est dilué dans une solution d'eau glucosée (glucose 5%) contenant 5% de crémophore et 3% de glycofurol.

Les composés selon l'invention sont administrés par gavage ou par infusion deux heures avant le prélèvement.

Le prélèvement sur citrate de sodium à 3,8% (1 volume pour 9 volumes de sang) est effectué par ponction à l'aorte abdominale après anesthésie de l'animal au pentobarbital sodique.

Le plasma riche en plaquettes (PRP) est obtenu par centrifugation du sang à 300g pendant 5 minutes, et les mesures d'agrégation plaquettaire sont réalisées comme décrites ci-dessus.

Les résultats sont calculés en utilisant l'aire sous la courbe d'absorbance mesurée pendant 6 minutes, et exprimés par le pourcentage (%) d'inhibition.

Le tableau III ci-après indique les résultats obtenus pour les composés 1 et 4 :

**TABLEAU III**

| Composés n° | Inhibition de l'agrégation plaquettaire *ex vivo* (sang rat) Dose = 3 mg/kg et t = 2 h après administration sauf spécification | |
|---|---|---|
| | Infusion (Intravenous) | Gavage (oral) |
| 1 | 79.0 + 5 .0 % (30 min) | 42.8 ± 10.7 % |
| 4 | 88.0 ± 5.0 % (1 mg/kg) | 72.0 ± 15.0 % |

Les composés de la présente invention sont notamment des principes actifs compatibles avec leur utilisation en tant que médicaments et/ou compositions pharmaceutiques.

Selon un de ces aspects, la présente invention concerne l'utilisation d'un composé de formule (I) ou d'un de ses sels, pharmaceutiquement acceptable pour la préparation de médicaments destinés à prévenir ou à traiter toute pathologie humaine et/ou à usage vétérinaire. Ainsi les composés selon l'invention peuvent être utilisés chez l'homme ou chez l'animal (notamment chez les mammifères incluant de façon non limitative les chiens, les chats, les chevaux, les bovins, les moutons) pour la prévention ou le traitement de maladies impliquant le récepteur P2Y12.

Ils sont donc indiqués en tant qu'inhibiteurs de l'activation plaquettaire, de l'agrégation et de la dégranulation plaquettaire, en tant que promoteurs de la désagrégation plaquettaire, et comme agents anti-thrombotiques. Ils sont également indiqués dans le traitement ou la prophylaxie d'angor (angine de poitrine) instable, d'angioplastie coronarienne percutanée (PTCA), d'infarctus du myocarde, de périthrombolyse, des complications artérielles thrombotiques de l'athérosclérose tels que les accidents vasculaires cérébraux emboliques ou thrombotiques, les accidents ischémiques transitoires, la maladie vasculaire périphérique, l'infarctus du myocarde avec ou sans thrombolyse, les complications artérielles de l'athérosclérose dues à des interventions chirurgicales comme l'angioplastie, l'endartériectomie, la pose de stent, les greffes vasculaires coronaires et autres, les complications thrombotiques de la chirurgie ou de dommages mécaniques comme la récupération de tissus après un traumatisme accidentel ou chirurgical, la chirurgie reconstructive (y compris la peau et les lambeaux musculaires), la coagulation intravasculaire disséminée, le purpura thrombotique thrombocytopénique, le syndrome hémolytique et urémique, les complications thrombotiques de la septicémie, le syndrome de détresse respiratoire, le syndrome des anti-phospholipides, la thrombocytopénie induite par l'héparine et la pré-éclampsie/éclampsie ; ou les thromboses veineuses telles que la thrombose veineuse profonde, la maladie veino-occlusive, les conditions hématologiques telles que la maladie myélo-proliférative (y compris la thrombocytémie), la drépanocytose; ou dans la prévention de l'activation plaquettaire induite mécaniquement *in vivo,* comme lors de la dérivation cardio-pulmonaire et de l'oxygénation extracorporelle (prévention des micro-thrombo-embolismes), dans la prévention de l'activation plaquettaire induite mécaniquement *in vitro* (utilisation dans la conservation des produits sanguins - par exemple, les concentrés plaquettaires - utilisation lors de dérivations telles que la dialyse rénale et la plasmaphérèse), la thrombose secondaire à une lésion vasculaire /inflammation tels que l'angéite, l'artérite, la glomérulonéphrite, la maladie inflammatoire de l'intestin, et le rejet de greffe d'organes, les conditions telles que la migraine, le phénomène de Raynaud, les conditions dans lesquelles les plaquettes peuvent contribuer au processus de la maladie inflammatoire sous-jacente dans la paroi vasculaire tel que la formation / progression de plaques athéromateuses, la sténose /resténose, et dans d'autres pathologies inflammatoires comme l'asthme, dans lequel les plaquettes et les facteurs dérivés des plaquettes sont impliqués dans le processus de la maladie immunologique.

L'utilisation des composés selon l'invention pour la prévention et/ou le traitement des maladies ci-dessus mentionnées, ainsi que pour la préparation de médicaments destinés à traiter ces maladies fait partie intégrante de l'invention.

Les composés de formule (I) ci-dessus, ou l'un de leurs sels pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Ainsi, selon un autre de ses aspects, la présente invention concerne des compositions pharmaceutiques renfermant, en tant que principe actif, un composé de formule (I), ou l'un de ses sels pharmaceutiquement acceptables ainsi qu'un ou plusieurs excipients pharmaceutiquement acceptables.

Dans les compositions pharmaceutiques de la présente invention pour l'administration par voie orale, sublinguale, inhalée, sous-cutanée, intramusculaire, intraveineuse, topique, locale, intratrachéale, intranasale, transdermique, locale ou rectale, les principes actifs peuvent être administrés sous formes unitaires d'administration, en mélange avec des supports pharmaceutiques classiques, aux animaux et aux êtres humains.

Les formes unitaires d'administration appropriées comprennent les formes par voie orale telles que les comprimés, les gélules molles ou dures, les poudres, les granules, les solutions ou suspensions orales, les formes d'administration sublinguale, buccale, intratrachéale, intraoculaire, intranasale, par inhalation, les aérosols, les formes d'administration topique, transdermique, les implants, les formes d'administration sous-cutanée, intramusculaire, intraveineuse et les formes d'administration rectale.

Pour l'administration topique on peut utiliser les composés selon l'invention dans des crèmes, des pommades, des gels, ou des lotions.

A titre d'exemple, une forme unitaire d'administration d'un composé selon l'invention sous forme de comprimé peut comprendre les composants suivants :

| | | |
|---|---|---|
| Composé selon l'invention | : | 50,0 mg |
| Mannitol | : | 223,75 mg |
| Croscarmellose sodique | : | 6,0 mg |
| Amidon de maïs | : | 15,0 mg |
| Hydroxypropyl-méthylcellulose | : | 2,25 mg |
| Stéarate de magnésium | : | 3,0 mg |

Par voie orale, la dose de principe actif administrée par jour peut atteindre 0,01 à 100 mg/kg, en une ou plusieurs prises, préférentiellement 0,02 à 50 mgHkg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou d'un de ses sels pharmaceutiquement acceptables.

Les composés selon l'invention pourront également être utilisés pour la préparation de compositions à usage vétérinaire.

## Revendications

1. Composé répondant à la formule (I) 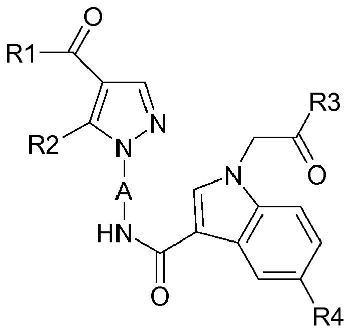 dans laquelle :
A représente un radical bivalent choisi parmi : 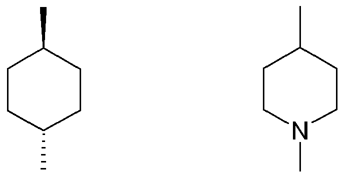 R₁ représente un (C₁-C₄)alkyle;
R₂ représente un (C₁-C₃)alkyle;
R₃ représente un groupe -NR₇R₈, R₇ et R₈, ensemble avec l'atome d'azote auquel ils sont liés constituent un hétérocycle saturé, comprenant de 4 à 6 chainons et pouvant contenir un autre atome d'azote ; ledit hétérocycle étant substitué par au moins un (C₁-C₃)alkyle non-substitué ou substitué par au moins un des groupes suivants:
• un, deux ou trois atomes d'halogène ou,
• un (C₁-C₃) alcoxy non-substitué ou substitué par un, deux ou trois atomes d'halogène;
R₄ représente un atome d'halogène,
à l'état de base ou de sel d'addition à un acide ou à une base ainsi que leurs énantiomères et diastéréoisomères, y compris leurs mélanges racémiques.

2. Composé de formule (I) selon la revendication 1, **caractérisé en ce que** A représente : 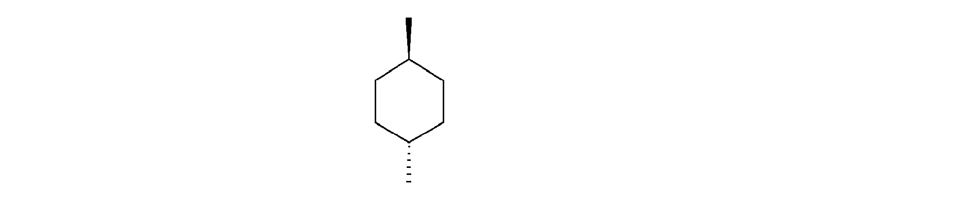 à l'état de base ou de sel d'addition à un acide ou à une base.

3. Composé de formule (I) selon la revendication 1, **caractérisé en ce que** A représente : 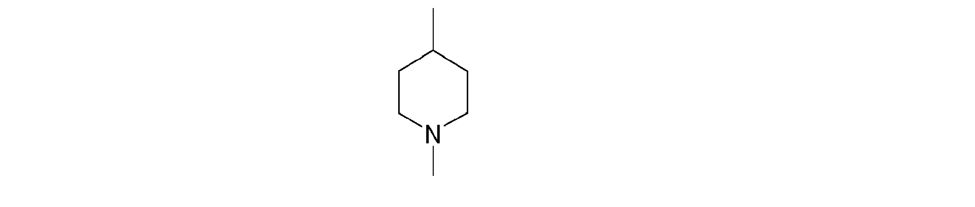 à l'état de base ou de sel d'addition à un acide ou à une base.

4. Composé de formule (I) selon l'une quelconque des revendications 1 à 3, **caractérisé en ce que** R₁ représente un groupe n-propyle, à l'état de base ou de sel d'addition à un acide ou à une base.

5. Composé de formule (I) selon l'une quelconque des revendications 1 à 4, **caractérisé en ce que** R₃ représente un groupe pipérazine substitué par au moins un (C₁-C₃)alkyle non-substitué ou substitué par au moins un des groupes suivants:
• un, deux ou trois atomes d'halogène ou,
• un (C₁-C₃) alcoxy non-substitué ou substitué par un, deux ou trois atomes d'halogène
à l'état de base ou de sel d'addition à un acide ou à une base.

6. Composé de formule (I) selon l'une des revendications 1 à 5, **caractérisé en ce que** R₄ représente un atome de chlore, à l'état de base ou de sel d'addition à un acide ou à une base.

7. Composé de formule (I) selon la revendication 1, **caractérisé en ce qu'**il est choisi parmi les composés suivants :
- 5-Chloro-1-[2-(4-methyl-piperazin-1-yl)-2-oxo-ethyl]-1H-indole-3-carboxylic acid [4-(4-butyryl-5-methyl-pyrazol-1-yl)-cyclohexyl]-amide
- 5-Chloro-1-[2-(4-methyl-piperazin-1-yl)-2-oxo-ethyl]-1H-indole-3-carboxylic acid [4-(4-butyryl-5-methyl-pyrazol-1-yl)-piperidin-1-yl]-amide
- 5-Chloro-1-{2-oxo-2-[4-(3,3,3-trifluoro-propyl)-piperazin-1-yl]-ethyl}-1H-indole-3-carboxylic acid [4-(4-butyryl-5-methyl-pyrazol-1-yl)-cyclohexyl]-amide
- 5-Chloro-1- {2-[4-(2-methoxy-ethyl)-piperazin-1-yl]-2-oxo-ethyl}-1H-indole-3-carboxylic acid [4-(4-butyryl-5-methyl-pyrazol-1-yl)-cyclohexyl]-amide
- 5-Chloro-1- {2-[4-(2-methoxy-ethyl)-piperazin-1-yl]-2-oxo-ethyl}-1H-indole-3-carboxylic acid [4-(4-butyryl-5-methyl-pyrazol-1-yl)-piperidin-1-yl]-amide
- 5-Chloro-1-{2-oxo-2-[4-(2-trifluoromethoxy-ethyl)-piperazin-1-yl]-ethyl}-1H-indole-3-carboxylic acid [4-(4-butyryl-5-methyl-pyrazol-1-yl)-piperidin-1-yl]-amide à l'état de base ou de sel d'addition à un acide ou à une base.

8. Procédé de préparation d'un composé de formule (I) selon l'une quelconque des revendications 1 à 7, **caractérisé en ce que** l'on fait réagir un composé de formule (IIB) : 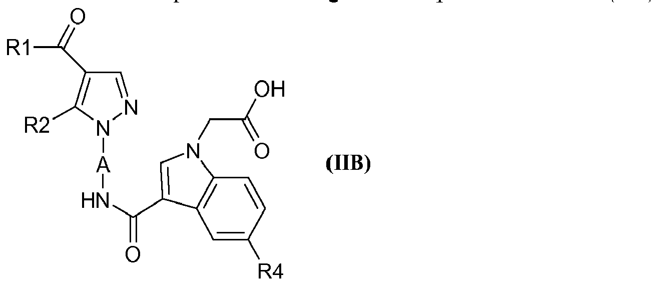 dans laquelle A, R₁, R₂ et R₄ sont tels que définis pour un composé de formule (I) à la revendication 1 avec une amine de formule (III) :
HR₃ **(III)**
dans laquelle R₃ est tel que défini pour un composé de formule (I) à la revendication 1.

9. Procédé de préparation d'un composé de formule (I) selon l'une quelconque des revendications 1 à 7, **caractérisé en ce que** l'on fait réagir un acide ou un dérivé fonctionnel activé de cet acide de formule : 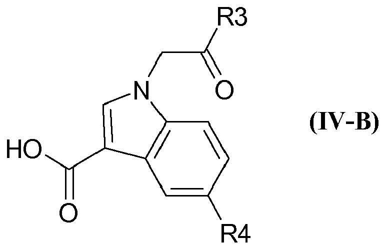 dans laquelle R₃ et R₄ sont tels que définis pour un composé de formule (I) à la revendication 1, avec un composé de formule : 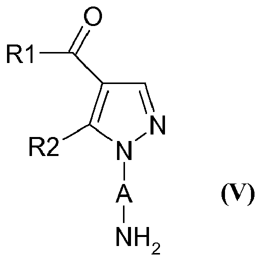 dans laquelle A, R₁ et R₂ sont tels que définis pour un composé de formule (I) à la revendication 1.

10. Composé de formule (IIA) : 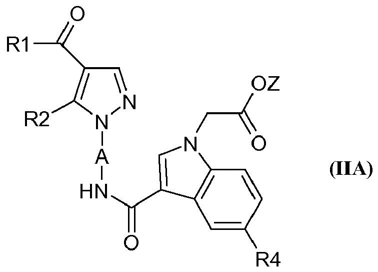 dans laquelle A, R₁, R₂ et R₄ sont tels que définis pour un composé de formule (I) à la revendication 1 et Z représente un (C₁-C₄) alkyle.

11. Composé de formule (IIB) : 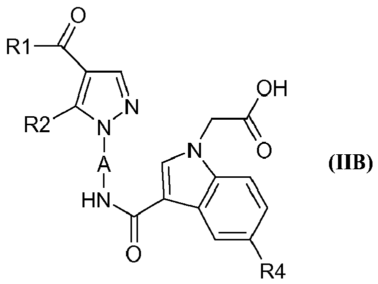 dans laquelle A, R₁, R₂ et R₄ sont tels que définis pour un composé de formule (I) à la revendication 1.

12. Médicament, **caractérisé en ce qu'**il comprend un composé de formule (I) selon l'une quelconque des revendications 1 à 7, ou un sel pharmaceutiquement acceptable du composé de formule (I).

13. Composition pharmaceutique **caractérisée en ce qu'**elle comprend un composé de formule (I) selon l'une quelconque des revendications 1 à 7, ou un sel pharmaceutiquement acceptable de ce composé, ainsi qu'au moins un excipient pharmaceutiquement acceptable.

14. Composé de formule (I) selon l'une quelconque des revendications 1 à 7, pour son utilisation dans le traitement ou la prophylaxie d'angor (angine de poitrine) instable, d'angioplastie coronarienne percutanée (PTCA), d'infarctus du myocarde, de péri-thrombolyse, des complications artérielles thrombotiques de l'athérosclérose tels que les accidents vasculaires cérébraux emboliques ou thrombotiques, les accidents ischémiques transitoires, la maladie vasculaire périphérique, l'infarctus du myocarde avec ou sans thrombolyse, les complications artérielles de l'athérosclérose dues à des interventions chirurgicales comme l'angioplastie, l'endartériectomie, la pose de stent, les greffes vasculaires coronaires et autres, les complications thrombotiques de la chirurgie ou de dommages mécaniques comme la récupération de tissus après un traumatisme accidentel ou chirurgical, la chirurgie reconstructive (y compris la peau et les lambeaux musculaires), la coagulation intravasculaire disséminée, le purpura thrombotique thrombocytopénique, le syndrome hémolytique et urémique, les complications thrombotiques de la septicémie, le syndrome de détresse respiratoire, le syndrome des anti-phospholipides, la thrombocytopénie induite par l'héparine et la pré-éclampsie/éclampsie ; ou les thromboses veineuses telles que la thrombose veineuse profonde, la maladie veino-occlusive, les conditions hématologiques telles que la maladie myélo-proliférative (y compris la thrombocytémie), la drépanocytose; ou dans la prévention de l'activation plaquettaire induite mécaniquement *in vivo,* comme lors de la dérivation cardio-pulmonaire et de l'oxygénation extracorporelle (prévention des micro-thrombo-embolismes), dans la prévention de l'activation plaquettaire induite mécaniquement *in vitro* (utilisation dans la conservation des produits sanguins - par exemple, les concentrés plaquettaires - utilisation lors de dérivations telles que la dialyse rénale et la plasmaphérèse), la thrombose secondaire à une lésion vasculaire /inflammation tels que l'angéite, l'artérite, la glomérulonéphrite, la maladie inflammatoire de l'intestin, et le rejet de greffe d'organes, les conditions telles que la migraine, le phénomène de Raynaud, les conditions dans lesquelles les plaquettes peuvent contribuer au processus de la maladie inflammatoire sous-jacente dans la paroi vasculaire tel que la formation / progression de plaques athéromateuses, la sténose /resténose, et dans d'autres pathologies inflammatoires comme l'asthme.
